(19) Europäisches Patentamt
European Patent Office
Office européen des brevets

(11) Veröffentlichungsnummer: **0 114 410**
**B1**

(12) # EUROPÄISCHE PATENTSCHRIFT

(45) Veröffentlichungstag der Patentschrift: **14.05.86**

(51) Int. Cl.⁴: **C 07 C 93/00**, A 61 K 31/13

(21) Anmeldenummer: **83113177.6**

(22) Anmeldetag: **28.12.83**

(54) **Substituierte alpha-(Äthyl)-alpha-(phenyl)-(omega-(dialkylamino)-alkoxy)-benzylalkohole, ihre Säureadditionssalze und quaternären Salze, Verfahren zu ihrer Herstellung sowie diese Verbindungen enthaltende Arzneimittel.**

(30) Priorität: **28.12.82 HU 419282**

(43) Veröffentlichungstag der Anmeldung: **01.08.84 Patentblatt 84/31**

(45) Bekanntmachung des Hinweises auf die Patenterteilung: **14.05.86 Patentblatt 86/20**

(84) Benannte Vertragsstaaten: **AT BE CH DE FR GB IT LI LU NL SE**

(56) Entgegenhaltungen:
**GB - A - 1 478 185**
**US - A - 3 075 014**

(73) Patentinhaber: **RICHTER GEDEON VEGYESZETI GYAR R.T., Gyömröi ut 19-21, H-1475 Budapest X (HU)**

(72) Erfinder: **Tóth, Edit, Szabolcska u. 7, H-1114 Budapest (HU)**
Erfinder: **Törley, József, Katona J. u. 41, H-1137 Budapest (HU)**
Erfinder: **Fekete, György, Dr., Széher u. 62, H-1021 Budapest (HU)**
Erfinder: **Szporny, László, Dr., Szabolcska u. 7, H-1114 Budapest (HU)**
Erfinder: **Vereczkey, László, Dr., Lajos u. 109, H-1036 Budapest (HU)**
Erfinder: **Pálosi, Eva, Dr., Vend u. 21, H-1025 Budapest (HU)**
Erfinder: **Klebovich, Imre, Dr., Karvaly u. 4, H-1125 Budapest (HU)**
Erfinder: **Vittay, Pál, Dr., József krt. 14, H-1085 Budapest (HU)**
Erfinder: **Görög, Sándor, Dr., Vajda P. u. 43, H-1089 Budapest (HU)**
Erfinder: **Hajdu, István, Tátra tér B/4, H-1205 Budapest (HU)**

(74) Vertreter: **Beszédes, Stephan G. Dr., Münchener Strasse 80a Postfach 1168, D-8060 Dachau (DE)**

EP 0 114 410 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue substituierte α-[Äthyl]-α-[phenyl]-[ω-(dialkylamino)-alkoxy]-benzylalkohole, ihre Säureadditionssalze und quaternären Salze, ein Verfahren zu ihrer Herstellung und diese Verbindungen enthaltende Arzneimittel.

Gegenstand der Erfindung sind substituierte α-[Äthyl]-α-[phenyl]-[ω-(dialkylamino)-alkoxy]-benzylalkohole der allgemeinen Formel

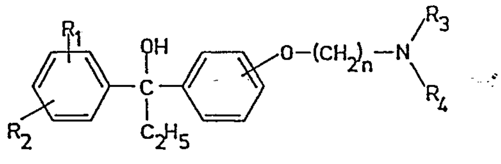

I,

worin

$R_1$ für ein Wasserstoffatom, ein Halogenatom, einen Trihalogenmethylrest oder einen geradkettigen oder verzweigten Alkyl oder Alkoxyrest mit je 1 bis 4 Kohlenstoffatom(en) steht,

$R_2$ ein Halogenatom, einen Trihalogenmethylrest oder einen geradkettigen oder verzweigten Alkyl- oder Alkoxyrest mit je 1 bis 4 Kohlenstoffatom(en) bedeutet,

$R_3$ und $R_4$ gleiche oder verschiedene geradkettige oder verzweigte Alkylreste mit 3 bis 5 Kohlenstoffatomen darstellen, und

n eine ganze Zahl von 2 bis 5 ist,

sowie ihre Säureadditionssalze und quaternären Ammoniumsalze.

Die Herstellung von Verbindungen ähnlicher Struktur ist in folgenden Literaturstellen beschrieben: C.A. *22*, 410[1]; *35*, 1781[2]; *40*, 4712[5]; *42*, P 1015 b; *47*, 9548 e; *50*, 12390 c; *50*, 2509 i; *55*, 17915 e; *55*, 15413 b; *75*, P 103682 b; *76*, P 119921 k; *82*, 16477 q; *90*, 86062 q; *92*, 52927 b. Nirgends wird jedoch eine pharmakologische Wirkung der hergestellten Verbindung erwähnt.

Ferner sind aus der britischen Patentschrift 1 478 185 gemäss deren Patentanspruch 1 substituierte Benzhydrolderivate, bei welchen der eine Benzolring unter anderen durch 1 oder mehr Halogenatom(e), Trihalogenmethylrest(e) und/oder geradkettige[n] und/oder verzweigte[n] gesättigte[n] und/oder ungesättigte[n] aliphatische[n] Kohlenwasserstoffrest(e) mit 1 bis 6 Kohlenstoffatom(en) substituiert sein kann, sowie ferner aus ihren Beispielen 13 und 14 4-(β-Diäthylamioäthoxy)-α-(äthyl)-benzhydrol und 4-(β-Diäthylaminoäthoxy)-α-(äthyl)-benzhydroläthobromid bekannt. Als pharmakologische Wirksamkeit dieser Verbindungen ist die Blockier- oder Induzierwirkung auf das Fremdsubstanzen umsetzende mikrosomale Enzymsystem angegeben. Für Substanzen, deren Inaktivierung durch die enzyminduzierende Wirkung der Verbindungen der britischen Patentschrift 1 478 185 beschleunigt wird, sind Bilirubin, Insektizide, Progesteron, Hexobarbital, Meprobamat und Bromsulfophthalein speziell erörtert.

Zwar können die Verbindungen der britischen Patentschrift 1 478 185 gemäss deren Patentanspruch 1 am einen Benzolring 1 oder mehr der Substituenten, welche $R_2$ in der Formel I der erfindungsgemässen Verbindungen bedeuten kann, aufweisen, die erfindungsgemässen Verbindungen sind jedoch im Gegensatz zu den Verbindungen der genannten Druckschrift, welche am zweiten Benzolring nicht substituiert sind, am zweiten Benzolring zwingend durch einen [ω-(Dialkylamino)-alkoxy]-rest substituiert.

Zwar haben die Verbindungen der Beispiele 13 und 14 der britischen Patentschrift 1 478 185, welche nicht unter deren Patentanspruch 1 fallen, einen β-Diäthylaminoäthoxysubstituenten, welcher den für den Fall, dass n in der Formel I der erfindungsgemässen Verbindungen 2 ist, in den erfindungsgemässen Verbindungen vorliegenden Dialkylaminoäthoxysubstituenten ähnlich ist, an ihrem einen Benzolring, auch von diesen bekannten Verbindungen, deren anderer Benzolring nicht substituiert ist, unterscheiden sich aber alle erfindungsgemässen Verbindungen darin, dass auch ihr anderer Benzolring substituiert ist.

Weiterhin sind in der US-Patentschrift 3 075 014 substituierte Diphenylbutanole beziehungsweise -butenole und entsprechende höhere Alkanole beziehungsweise Alkenole beschrieben, wobei als niedrigste spezielle Alkanole Pentanole genannt sind. Als spezielle Indikationen ihrer pharmakologischen Wirkung sind die Behandlung von Hypercholesterinämie und Herzgefässerkrankungen, welche durch hohe Cholesterinblutspiegel verschärft werden, sowie entzündungshemmende, östrogene und antiöstrogene Wirkungen angegeben.

Die erfindungsgemässen Verbindungen unterscheiden sich von allen Verbindungen der US-Patentschrift 3 075 014 darin, dass die letzteren mindestens Butanole sind, während die erfindungsgemässen Verbindungen zwingend Propanole sind.

Vorzugsweise ist, beziehungsweise sind, das, beziehungsweise die, Halogenatom(e), für das, beziehungsweise die, $R_1$ und/oder $R_2$ stehen kann, beziehungsweise können, [ein] Chlor-, Fluor- und/oder Bromatom(e).

Es ist auch bevorzugt, dass der, beziehungsweise die, Trihalogenmethylrest(e), für den, beziehungsweise die, $R_1$ und/oder $R_2$ stehen kann, beziehungsweise können, [ein] Trifluormethylrest(e) und/oder Trichlormethylrest(e), insbesondere der, beziehungsweise die, erstere(n), ist, beziehungsweise sind.

Ferner ist es bevorzugt, dass der, beziehungsweise die, Alkyl- und/oder Alkoxyrest(e), für den, beziehungsweise die, $R_1$ und/oder $R_2$ stehen kann, beziehungsweise können, ein solcher, beziehungsweise solche, mit 1 oder 2, insbesondere 1, Kohlenstoffatom(en) ist, beziehungsweise sind.

Weiterhin ist es bevorzugt, dass die Alkylreste, für die $R_3$ und $R_4$ stehen, solche mit 3 oder 4, insbesondere 3, Kohlenstoffatomen sind.

Ausserdem ist es bevorzugt, dass n 2 bis 4, insbesondere 2 oder 3, ist.

Bevorzugt ist der substituierte [ω-(Dialkylamino)-alkoxy]-rest in der 4-Stellung des Benzolringes, an welchem er hängt.

Besonders bevorzugte erfindungsgemässe α-[Äthyl]-α-[phenyl]-ω-(dialkylamino)-alkoxy]-benzylalkohole sind α-Äthyl-α-(2-methoxyphenyl)-4-[2-(diisopropylamino)-äthoxy]-benzylalkohol, α-Äthyl-α-(4-fluorphenyl)-4-[3-(di-n-propylamino)-propoxy]-benzylalkohol, α-Äthyl-α-(4-chlorphenyl)-4-[3-(di-n-propylamino)-propoxy]-benzylalkohol, α-Äthyl-α-(2,4-dichlorphenyl)-4-[3-(di-n-propylamino)-propoxy]-benzylalkohol, α-Äthyl-α-(4-bromphenyl)-4-[3-(di-n-propylamino)-propoxy]-benzylalkohol, α-Äthyl-α-(3-chlorphenyl)-4-[3-(di-n-propylamino)-propoxy]-benzylalkohol, α-Äthyl-α-(3-trifluormethylphenyl)-4-[3-(di-n-propylamino)-propoxy]-benzylalkohol, α-Äthyl-α-(3-trifluormethylphenyl)-4-[2-(diisopropylamino)-äthoxy]-benzylalkohol, α-Äthyl-α-(4-chlorphenyl)-4-[2-(diisopropylamino)-äthoxy]-benzylalkohol, α-Äthyl-α-(2-trifluormethylphenyl)-4-[2-(diisopropylamino)-äthoxy]-benzylalkohol, α-Äthyl-α-(2-methoxyphenyl)-2-[2-(diisopropylamino)-äthoxy]-benzylalkohol, α-Äthyl-α-(4-fluorphenyl)-4-[2-(diisopropylamino)-äthoxy]-benzylalkohol, α-Äthyl-α-(2,5-dimethylphenyl)-4-[2-diisopropylamino)-äthoxy]-benzylalkohol, α-Äthyl-α-(3-chlorphenyl)-4-[2-(diisopropylamino)-äthoxy]-benzylalkohol und α-Äthyl-α-(2-methoxyphenyl)-4-[3-(di-n-propylamino)-propoxy]-benzylalkohol sowie ihre Salze.

Gegenstand der Erfindung ist auch ein Verfahren zur Herstellung der erfindungsgemässen Verbindungen, welches dadurch gekennzeichnet ist, dass

a) am Benzolring ω-(dialkylamino)-alkoxysubstituierte Propiophenone der allgemeinen Formel

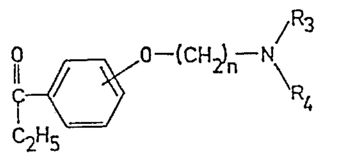
II,

worin $R_3$, $R_4$ und n wie oben festgelegt sind, mit eine Phenylgruppe aufweisenden metallorganischen Verbindungen der allgemeinen Formel

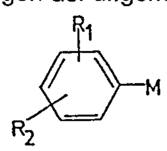
III,

worin

$R_1$ und $R_2$ wie oben festgelegt sind, und

M für ein Alkalimetallatom, vorzugsweise Lithium, Natrium- oder Kaliumatom, oder einen Rest der Formel MgX mit X = Halogenatom steht,

umgesetzt werden, oder

b) substituierte α-[Äthyl]-α-[phenyl]-[ω-(halogen)-alkoxy]-benzylalkohole der allgemeinen Formel

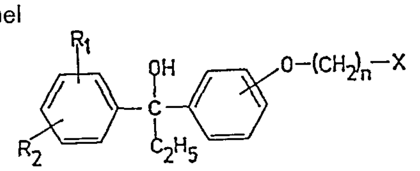
IV,

worin

$R_1$, $R_2$ und n wie oben festgelegt sind, und

X für ein Halogenatom steht,

mit Dialkylaminen der allgemeinen Formel

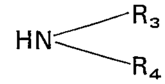
V,

worin $R_3$ und $R_4$ wie oben festgelegt sind, vorzugsweise in Gegenwart von Basen, umgesetzt werden oder

c) substituierte [ω-(Dialkylamino)-alkoxy]-benzophenone der allgemeinen Formel

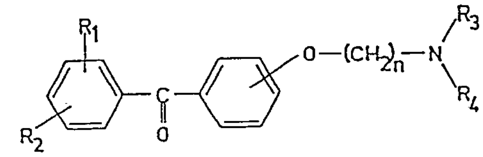
VI,

worin $R_1$, $R_2$, $R_3$, $R_4$ und n wie oben festgelegt sind, mit eine Äthylgruppe aufweisenden metallorganischen Verbindungen, insbesondere Äthylmagnesiumhalogeniden oder Äthyllithium, umgesetzt werden, oder

d) am Benzolring substituierte Propiophenone der allgemeinen Formel

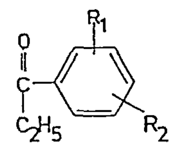
VII,

worin $R_1$ und $R_2$ wie oben festgelegt sind, mit eine {[ω-(Dialkylamino)-alkoxy]-phenyl}-gruppe aufweisenden Grignard-Verbindungen der allgemeinen Formel

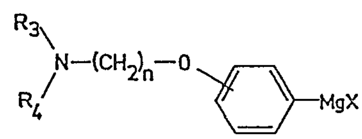
VIII,

worin

$R_3$, $R_4$ und n wie oben festgelegt sind, und

X für ein Halogenatom steht,

umgesetzt werden, oder

e) substituierte 1-[Phenyl]-1-[hydroxyphenyl]-propan-1-ole der allgemeinen Formel

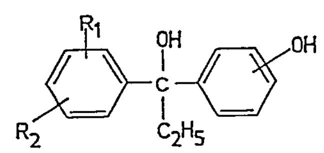
IX,

worin $R_1$ und $R_2$ wie oben festgelegt sind, vorzugsweise in Form ihrer Alkaliphenolate oder quaternären Ammoniumphenolate, mit tertiären Aminen der allgemeinen Formel

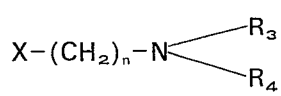
X,

worin

$R_3$, $R_4$ und n wie oben festgelegt sind, und

X für ein Halogenatom oder einen Alkylsulfonyloxy- oder Arylsulfonyloxyrest steht,

oder mit deren Salzen, vorzugsweise in Gegenwart

von säurebindenden Mitteln, umgesetzt werden, oder

f) substituierte α-[Äthinyl]- beziehungsweise α-[Vinyl]-α-[phenyl]-[ω-(dialkylamino)-alkoxy]-benzylalkohole der allgemeinen Formel

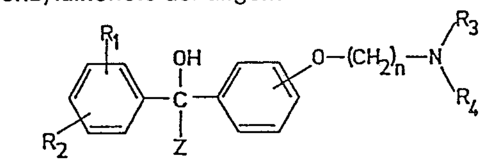

XI,

worin

$R_1$, $R_2$, $R_3$, $R_4$ und n wie oben festgelegt sind, und

Z für einen Äthinyl- oder Vinylrest steht,

reduziert werden,

worauf gegebenenfalls in an sich bekannter Weise die erhaltenen α-[Äthyl]-α-[phenyl]-[ω-(dialkylamino)-alkoxy]-benzylalkohole der allgemeinen Formel I mit Säuren in Säureadditionssalze oder mit Quaternisierungsmitteln in quaternäre Ammoniumsalze überführt werden oder gegenbenenfalls die erhaltenen Säureadditionssalze der α-[Äthyl]-α-[phenyl]-[ω-(dialkylamino)-alkoxy]-benzylalkohole der allgemeinen Formel I in die entsprechenden freien Basen der allgemeinen Formel I und/oder in andere Säureadditionssalze überführt werden.

Gemäss einer bevorzugten Ausführungsform der Variante a) des erfindungsgemässen Verfahrens setzt man die Propiophenone der allgemeinen Formel (II) in einem wasserfreien, inerten organischen Lösungsmittel mit der metallorganischen Verbindung der allgemeinen Formel (III), vorzugsweise dem entsprechend substituierten Phenylmagnesiumchlorid oder -bromid oder dem entsprechend substituierten Phenyllithium, um. Als Lösungsmittel verwendet man aprotische organische Lösungsmittel, zum Beispiel die folgenden: aliphatische Äther, wie Diäthyläther, Di-n-butyläther, Diäthylenglykoldimethyläther, oder alicyclische Äther, wie Tetrahydrofuran oder Dioxan, aliphatische oder aromatische Kohlenwasserstoffe, zum Beispiel Ligroin, Benzol, Toluol oder Xylol, ferner Dimethylsulfoxyd, Hexamethylphosphoramid oder Gemische der aufgeführten Lösungsmittel. Die metallorganische Verbindung wird in wenigstens äquimolarer Menge eingesetzt. Die Reaktion wird vorzugsweise unter Schutzgsatmosphäre, zum Beispiel unter Stickstoff- oder Argonatmosphäre, vorgenommen. Die Reaktionstemperatur liegt zwischen −60° C und dem Siedepunkt des Lösungsmittels, vorzugsweise zwischen −30 und 100° C. Nach Beendigung der Reaktion wird das metallorganische Zwischenprodukt zweckmässig mit einer Ammoniumchloridlösung hydrolytisch gespalten und die gebildete Verbindung der allgemeinen Formel (I) isoliert. Das Produkt kann in an sich bekannter Weise, zum Beispiel durch Destillieren oder Umkristallisieren, gereinigt werden.

Gemäss der Verfahrensvariante b) werden die Verbindungen der allgemeinen Formel (IV), in denen X vorzugsweise für ein Chlor- oder Bromatom steht, mit sekundären Aminen der allgemeinen Formel (V) umgesetzt. Die Umsetzung wird vorzugsweise in einem organischen Lösungsmittel sowie in Gegenwart eines Säureakzeptors vorgenommen. Als Lösungsmittel kommen zum Beispiel in Betracht: Kohlenwasserstoffe, wie Ligroin, Benzol, Toluol, oder halogenierte Kohlenwasserstoffe, wie Chloroform, Äther, zum Beispiel Dioxan, Alkohole, wie Äthanol, Ketone, zum Beispiel Aceton oder Methylisobutylketon, Ester, wie Äthylacetat, Säureamide, zum Beispiel Dimethylformamid, oder Gemische der aufgeführten Lösungsmittel. Als Säureakzeptor verwendet man vorzugsweise anorganische oder tertiäre organische Basen, insbesondere Amine, oder aber einen Überschuss des Amins der allgemeinen Formel (V). Wird als Säureakzeptor eine tertiäre organische Base oder der Überschuss des Amins der allgemeinen Formel (V) verwendet, so können diese gleichzeitig als Lösungsmittel wirken. Die Reaktionstemperatur liegt zwischen 20° C und dem Siedepunkt des Lösungsmittels. Nach Beendigung der Reaktion wird das Produkt abgetrennt. Zur Aufarbeitung giesst man das Reaktionsgemisch beispielsweise in Wasser und extrahiert mit einem organischen Lösungsmittel. Die organische Phase wird mit Wasser halogenfrei gewaschen, getrocknet und dann eingedampft. Das Rohprodukt kann durch Destillieren oder Umkristallisieren gereinigt werden.

Gemäss der Verfahrensvariante c) werden die Benzophenone der allgemeinen Formel (VI) mit wenigstens der äquimolaren Menge von vorzugsweise Äthylmagnesiumbromid oder -jodid oder von Äthyllithium umgesetzt. Die Umsetzung wird ähnlich wie die Umsetzung gemäss a) in einem inerten, wasserfreien organischen Lösungsmittel vorgenommen.

Gemäss der Verfahrensvariante d) werden Grignard-Verbindungen der allgemeinen Formel (VIII), insbesondere solche, in denen X für ein Bromatom steht, in einem wasserfreien, inerten organischen Lösungsmittel mit wenigstens der äquimolaren Menge des Propiophenons der allgemeinen Formel (VII) ähnlich wie unter a) beschrieben umgesetzt.

Gemäss der Verfahrensvariante e) werden Verbindungen der allgemeinen Formel (IX), vorzugsweise in Form ihrer Alkaliphenolate oder quaternären Ammoniumphenolate, mit tertiären Aminen der allgemeinen Formel (X) kondensiert. Als tertiäre Amine kommen zum Beispiel Dialkylaminoalkylmesylate, -tosylate, -bromide und insbesondere -chloride in Frage, gegebenenfalls in Form eines Hydrohalogenides. Die Reaktion wird vorzugsweise in Gegenwart eines Säureakzeptors in einem wasserfreien oder wasserhaltigen organischen Lösungsmittel vorgenommen. Als Lösungsmittel kommen in Frage: Ester, wie Äthylacetat; Äther, wie Dioxan, Tetrahydrofuran oder Diäthyläther; Kohlenwasserstoffe, zum Beispiel Ligroin, Benzol, Toluol, Xylol; halogenierte Kohlenwasserstoffe, wie Chloroform oder Chlorbenzol; Säureamide, wie Dimethylformamid; Ketone, zum Beispiel Aceton, Methyläthylketon, Methylisobutylketon; Alkohole, wie Äthanol oder Propanol. Die Phenolate der Verbindungen der allgemeinen Formel (IX) können in an sich bekannter Weise gebildet werden, zum Beispiel durch Umsetzen mit Alkalialkoholaten, -amiden, -hydriden, -hydroxyden, -carbonaten oder quaternären Ammoniumverbindungen. Als Säureakzeptor kommen vorzugsweise anorganische oder tertiäre organische Basen, zum Beispiel Natriumhydroxyd, Kaliumhydroxyd, Kaliumcarbonat, Triäthylamin und Pyridin, in Betracht. Die Umsetzung kann in Gegenwart eines Katalysators vorgenommen werden. Als Katalysator kann zum Beispiel ein Alkalihalogenid, vorzugsweise ein Alkalijodid, eingesetzt werden. Die Reaktionstemperatur kann innerhalb weiter Grenzen variieren, im allgemeinen arbeitet man bei Temperaturen zwischen 20° C und dem Siedepunkt des Lösungsmittels.

Gemäss einer bevorzugten Ausführungsform der Verfahrensvariante f) wird die Reduktion der Äthinyl- beziehungsweise Vinylverbindungen der allgemeinen Formel (XI) durch katalytische Hydrieren vorgenommen. Als Hydrierkatalysator werden Metalle, zum Beispiel Ruthenium, Palladium, Platin, Nickel, Eisen, Kupfer, Kobalt, Chrom, Zink, Molybdän oder Wolfram, oder deren Oxyde oder Sulfide verwendet. Der Katalysator kann zum Beispiel hergestellt werden, indem man das Oxyd des betreffenden Metalls unmittelbar im Reaktionsgefäss mit Wasserstoff reduziert. Diese Methode wird dann angewandt, wenn man als Katalysator feinverteiltes Platin oder Palladium verwenden will. Man kann auch Katalysatoren verwenden, die auf einen Träger aufgebracht wurden. Geeignete Träger sind zum Beispiel Tierkohle, Siliciumdioxyd, Aluminiumoxyd sowie die Sulfate und Carbonate der Erdalkalimetalle. Die Reduktion kann auch in Gegenwart von Raney-Nickel vorgenommen werden. Besonders bevorzugt sind als Katalysator Palladium, zweckmässig in Form von Palladiumaktivkohle, und Raney-Nickel. Die Reduktion wird in einem inerten organischen Lösungsmittel, zum Beispiel niederen aliphatischen Alkohlen, Äthern, Ester, aliphatischen, cycloaliphatischen oder aromatischen Kohlenwasserstoffen oder in deren Gemischen, vorgenommen. Man kann bei atmosphärischem Druck oder unter erhöhtem Druck hydrieren, bevorzugt ist ein Druck von weniger als 506 kPa. Die Temperatur liegt zwischen 20° C und dem Siedepunkt des Lösungsmittels. Bevorzugt wird bei Raumtemperatur unter atmosphärischem Druck bis zur Beendigung der Wasserstoffaufnahme hydriert. Dann wird der Katalysator abfiltriert, das Filtrat eingedampft und das Produkt zum Beispiel durch Destillation oder Umkristallisation gereinigt.

Aus den Verbindungen der allgemeinen Formel (I) können gewünschtenfalls in an sich bekannter Weise Säureadditionssalze oder quaternäre Ammoniumsalze hergestellt werden. Zur Bildung der Säureadditionssalze sind anorganische und organische Säuren geeignet, zum Beispiel: Halogenwasserstoffsäuren, wie Salzsäure oder Bromwasserstoffsäure, Schwefelsäure, Phosphorsäure, Ameisensäure, Essigsäure, Propionsäure, Oxalsäure, Glykolsäure, Maleinsäure, Fumarsäure, Bernsteinsäure, Weinsäure, Ascorbinsäure, Citronensäure, Apfelsäure, Salicylsäure, Milchsäure, Benzoesäure, Zimtsäure, Asparaginsäure, Glutaminsäure, N-Acetylasparaginsäure, N-Acetylglutaminsäure, Alkylsulfonsäuren, zum Beispiel Methansulfonsäure, Arylsulfonsäuren, wie p-Toluolsulfonsäure.

Zur Salzbildung löst man zum Beispiel die Verbindung der allgemeinen Formel (I) in einem inerten Lösungsmittel, zum Beispiel in Äthanol, und gibt zu der Lösung die entsprechende Säure. Das gebildete Salz fällt man zweckmässig mit einem mit Wasser nicht mischbaren Lösungsmittel, zum Beispiel mit Diäthyläther, aus. Zur Bildung der quaternären Salze verwendet man zweckmässig niedere Alkyl-, Alkenyl- oder Benzylhalogenide oder Alkylsulfate. Die Salzbildung erfolgt in einem organischen Lösungsmittel, zweckmässig in Aceton, Acetonitril, Äthanol oder in Gemischen der genannten Lösungsmittel, bei Temperaturen zwischen Raumtemperatur und der Siedetemperatur des Lösungsmittels. Das quaternäre Salz kann zum Beispiel durch Filtrieren abgetrennt und notwendigenfalls durch Umkristallisieren gereinigt werden.

Die Ausgangsverbindungen sind bekannt beziehungsweise können nach literaturbekannten Verfahren hergestellt werden. Die Ketone der allgemeinen Formeln (II), (VI) und (VII) können zum Beispiel mit der Friedel-Crafts-Ketonsynthese erhalten werden (G.A. Olah: Friedel-Crafts and related reactions, Band III/1, Ed.: Interscience Publishers, 1964, S. 1-63).

Die Verbindungen der allgemeinen Formel (III) und (VIII) erhält man zum Beispiel, indem man aus den entsprechend substituierten Arylhalogeniden in an sich bekannter Weise das Grignard-Reagens bildet (M.S. Kharash et al.: Grignard reactions of nonmetallic substances, Ed. Prentice-Hall. Inc., 1954, S. 5-90). Die ein Alkalimetall enthaltenden metallorganischen Verbindungen können zum Beispiel nach Houben-Weyl: Methoden der organischen Chemie, Bd. XIII/1, S. 134-159 sowie 389-405 (1970) hergestellt werden.

Die Verbindungen der allgemeinen Formeln (IV) und (IX) sind zum Beispiel durch Umsetzen der entsprechend substituierten Propiophenone mit entsprechenden Grignard-Reagentien erhältlich (s. zum Beispiel das oben zitierte Buch von M.S. Kharash et al., S. 138-143).

Zur Herstellung der Verbindungen der allgemeinen Formel (XI) setzt man – für den Fall, dass Z für einen Vinylrest steht – Benzophenone der allgemeinen Formel (VI) mit einem Vinylmagnesiumhalogenid um, und für den Fall, dass Z für einen Äthinylrest steht, äthinyliert man das Benzophenon der allgemeinen Formel (VI), zum Beispiel auf die in der ungarischen Patentschrift Nr. 166 769 beschriebene Weise.

Die Verbindungen der allgemeinen Formel (I) sind zur Behandlung akuter Äthanolintoxikation geeignet und für diesen Zweck ausgedehnt verwendbar. Die akute Alkoholintoxikation ist vor allem durch Euphorie, allgemeine Stimuliertheit, Ataxie, Somnolenz und paralytische Zustände

charakterisiert. Die Gefahren dieses toxishen, krankhaften Zustandes sind bekannt und nicht zu vernachlässigen. Die intoxierte Person gefährdet ihre Umgebung (zum Beispiel Trunkenkeit am Steuer) und sich selbst.

Die akute Alkoholintoxikation ist für den ischämischen Gehirninfarkt ein bedeutender Risikofaktor (Hillbom, M. u. a.: Lancet *2,* 1181 [1978]; Stroke *12,* 422 [1981]). Für den alkoholintoxierten Zustand gibt es kein geeignetes Antidotum. Die durch Alkohol ausgelöste lokomotorische Hyperaktivität wird an Mäusen durch α-Methyl-parathyrosin in einem Dosisbereich normalisiert, in dem die Verbindung die spontane lokomotorische Aktivität der Tiere beeinträchtigt (Carlsson, A. u.a.: Psychopharm. *26,* 307, 1972). Die narkotisierende Wirkung des Alkohols wird durch Stimulantien (Coffein, Amphetamin) verringert, die motorische Inkoordination (Ataxie) jedoch gleichzeitig prolongiert (Wallagsen, H. u. a.: Actions of alcohol, Amsterdam, Elsevier, 1970; Rech, R.H. u. a.: Ann. N.Y. Acad. Sci. *28,* 426, 1976; Todzy, I. u. a.: Psychopharm. *59,* 143, 1978). Die alkoholische Intoxikation, die Narkose wird durch L-Cystein verkürzt (Sprince, H. u. a.: Agents and Actions, *4,* 125, 1974; Nagasawa, H.T. u. a.: Life Sci., *17,* 707, 1975); L-Cystein wurde zu den folgenden Untersuchungen über Schlaf unter Alkoholeinfluss als Referenzsubstanz verwendet.

Da Alkohol keine Substanz ist, welche durch Induzierung der Wirkung eines Enzymes schneller inaktiviert werden kann, hat die pharmakologische Wirksamkeit der Verbindungen der britischen Patentschrift 1 478 185 mit den pharmakologischen Wirkungen gegen akute Alkoholintoxikation der erfindungsgemässen Verbindung nichts zu tun.

Auch haben die Verbindungen der genannten Druckschrift überhaupt keine Wirkung gegen akute Alkoholintoxikation.

Zur Messung der Änderung der Dauer der durch Alkohol hervorgerufenen Narkose wurden 16 Stunden lang ausgehungerte, 160-180 g schwere Hann.-Wistar-Ratten beiderlei Geschlechts verwendet. Jede Gruppe bestand aus 10 Tieren, die mit entsprechenden Dosen der Testverbindungen oral behandelt wurden. Eine Stunde nach der Behandlung wurde den Tieren in einer Dosis von 3,5 g/kg intraperitoneal Äthanol appliziert. Die Schlafdauer der Tiere wurde vom Ausfall des Aufrichtreflexes (Righting Reflexes) bis zur spontanen Korrektur der Körperhaltung gemessen. Für jede Gruppe wurde die durchschnittliche Schlafzeit, die Standardabweichung und das Ergebnis in Prozent der Kontrolle berechnet. Die Kontrolle erhielt Placebo und ebenfalls 3,5 g/kg Alkohol. Die Ergebnisse sind in der folgenden Tabelle 1 angegeben, in der $\bar{x} \pm$S.E. Durchschnittswert $\pm$ Standardabweichung bedeutet. Die Schlafdauer der Kontrolle betrug 88,5 $\pm$ 3,54 min.

A = α-Äthyl-α-(2-methoxyphenyl)-4-[3-(di-n-propylamino)-propoxy]-benzylalkohol

B = α-Äthyl-α-(3-chlorphenyl)-4-[3-(di-n-propylamino)-propoxy]-benzylalkohol

C = α-Äthyl-α-(2-trifluormethylphenyl)-4-[3-(diisopropylamino)-äthoxy]-benzylalkohol

*Tabelle 1*

| Behandlung | Dosis (mg/kg) | Schlafdauer in % der Kontrolle ±S.E. |
|---|---|---|
| Kontrolle | | 100± 4,0 |
| A | 5,0 | 60±10,1 |
| | 10,0 | 50± 6,9 |
| B | 0,3 | 65± 6,8 |
| | 1,0 | 53± 4,7 |
| | 10,0 | 49± 2,8 |
| C | 40,0 | 62± 5,1 |
| L-Cystein | 500,0 | 63± 4,2 |

Aus den Ergebnissen ist ersichtliche, dass die Verbindungen der allgemeinen Formel (I) die deprimierende Wirkung des Alkohols auf das Zentralnervensystem bedeutend verringern, die Narkosedauer wesentlich verkürzen. In Dosen, die um 1-3 Grössenordnungen geringer sind als die des L-Cysteins, erreichen beziehungsweise übertreffen sie dessen verkürzende Wirkung auf die Dauer der Narkose. Äthanol stimuliert in von der Dosis abhängender Weise das Zentralnervensystem, löst Hyperaktivität aus.

Die Wirkung der erfinsungdgemässen Verbindungen auf die durch Alkohol ausgelöste lokomotorische Hyperaktivität wurde an 16-18 g schweren BALB/c-Mäusen beiderlei Geschlechts untersucht. Die aus je 15 Tieren bestehenden Gruppen erhielten die Testverbindungen in einer Dosis von 40 mg/kg oral, und eine Stunde später intraperitoneal Placebo beziehungsweise 2 g/kg Äthanol. Die Kontrollgruppe wurde mit Placebo behandelt. Die lokomotorische Aktivität der Tiere wurde mit einem Motimeter des Typs Animex B.S.E. zwei Stunden lang gemessen. Die Ergebnisse sind in Prozent der Kontrolle in der Tabelle 2 zusammengefasst.

*Tabelle 2*

| Behandlung | Dosis Verbindung | (mg/kg) Alkohol | Lokomotorische Aktivität (% Kontr.) |
|---|---|---|---|
| Placebo-Kontr. | — | — | 100± 9,8 |
| Äthanol + Placebo | | 2000,0 | 170±11,8 |
| B + Placebo | 40 | | 108±13,3 |
| B + Äthanol | 40 | 2000,0 | 70± 8,4 |

Placebo-Kontrolle $\bar{x} \pm$S.E. = 3118,3 $\pm$305,6 Bewegungen/2 Stunden

Aus der Tabelle 2 ist ersichtlich, dass die erfindungsgemässen Verbindungen die durch Alkohol ausgelöste gesteigerte lokomotorische Aktivität bedeutend verringern, die Verbindungen selbst jedoch die spontane lokomotorische Aktivität der Tiere nicht verändern.

Die akute Toxizität der Verbindungen der allgemeinen Formel (I) wurde an 160-180 g schweren Wister-Ratten beiderlei Geschlechts untersucht. Jede Gruppe bestand aus 10 Tieren, die mit je 500 mg/kg der Testverbindung in einmaliger Dosis oral behandelt wurden. Die Tiere wurden 14 Tage lang beobachtet. Von den mit den Verbindungen A, B und C und von den mit L-Cystein behandelten Tieren verendete kein einziges. Daraus ist ersichtlich, dass die Toxizität der erfindungsgemässen Verbindungen, bezogen auf die wirksame Dosis, sehr günstig ist.

Durch Untersuchungen auf die im folgenden angegebene Weise wurde festgestellt, dass die Verbindungen auch in einer Dosis von 160 mg/kg keinerlei sonstige Wirkung auf das Zentralnervensystem ausüben: Elektroschock (Swinyard, E.A., Brown, W.C., Goodman, L.S.: J. Pharmacol. Exp. Ther, *106*, 319 [1952]), Metrazolkrampf (Everett, G.M., Richards, R.K.: J. Pharmacol. Exp. Ther., *81*, 402 [1944]), Thiosemicarbazidkrampf (Da Vanzo, J.P., Greig, M.E., Cormin, M.A.: Amer. J. Physiol., *201*, 833 [1961]), Strychninkrampf (Kerley, T.L., Richards, A.G., Begley, R.W., Abreu, B.B., Wesver, L.C.: J. Pharmacol. Exp. Ther., *132*, 360 [1961]), Nikotinkrampf (Stone, C.A., Mecklenburg, K.L., Torhans, M.L.: Arch. Int. Pharmacodyn., *117*, 419 [1958]), Drehstab (Kinnard, W.J., Carr., C.J.: J. Pharmacol. Exp. Ther., *121*, 354 [1957]), Physostigminletalität (Nose, T. und Kojima, M.: Europ. J. Pharmacol., *10*, 83 [1970]), yohimbinpotenzierende Wirkung (Quinton, R.M.: Brit. J. Pharmacol., *21*, 51 [1963]), schmerzstillende Wirkung (Bianchi, C., Franceschini, J.: Brit. J. Pharm. Chemother., *9*, 280 [1954]).

Die erfindungsgemässen Verbindungen können zu Arzneimittelpräparaten zubereitet werden. Die Präparate können oral, rektal und/oder parenteral verabreicht werden. Zur oralen Darreichung können Tabletten, Dragees oder Kapseln hergestellt werden. Bei der Herstellung von oral verabreichbaren Formen werden als Streckmittel zum Beispiel Milchzucker oder Stärke verwendet. Als Binde- oder Granuliermittel kommen zum Beispiel Gelatine, Carboxymethylcellulose-natrium, Methylcellulose, Polyvinylpyrrolidon oder Stärkekleister in Betracht. Als Sprengmittel werden in erster Linie Kartoffelstärke oder mikrokristalline Cellulose zugesetzt, jedoch können beispielsweise auch Ultraamylopektin oder Formaldehyd casein verwendet werden. Als Antihaftmittel und Gleitmittel kommen z. B. Talk, kolloidale Kieselsäure, Stearin, Ca- und Mg-Stearat in Frage.

Die Tabletten können zum Beispiel durch Nassgranulieren und anschliessendes Pressen hergestellt werden. Das Gemisch aus Wirkstoff und Füllstoffen sowie gegebenenfalls ein Teil des Sprengmittels werden mit der wässerigen, alkoholischen oder wässerig-alkoholischen Lösung der Bindemittel in einer geeigneten Vorrichtung granuliert, und das Granulat wird getrocknet. Zu dem trockenen Granulat werden der Rest des Sprengmittels, ferner die Antihaftmittel und das Gleitmittel gegeben, und das Gemisch wird zu Tabletten gepresst. Gegebenenfalls können die Tabletten zur Erleichterung der Dosierung mit Teilungsmarkierungen versehen werden. Die Tabletten können auch unmittelbar durch Pressen eines Gemisches aus dem Wirkstoff und geeigneten Hilfsstoffen hergestellt werden.

Die Tabletten können gewünschtenfalls unter Verwendung der in der Arzneimittelherstellung üblichen Schutz-, Geschmacks- und Farbstoffe, zum Beispiel Zucker, Cellulosederivate, wie Methyl- oder Äthylcellulose oder Carboxymethylcellulosenatrium, Polyvinylpyrrolidon, Calciumphosphat, Calciumcarbonat, Lebensmittelfarbstoffe, Lebensmittelfarblacke, Aromastoffe und Eisenoxydpigmente, auf die übliche Weise dragiert werden.

Zur Herstellung von Kapseln wird das Gemisch aus Wirkstoff und Hilfsstoffen in Kapseln gefüllt.

Zur rektalen Anwendung werden Suppositorien hergestellt. Diese enthalten ausser dem Wirkstoff eine Trägermasse, das sog. Adeps pro suppositori. Als dieses kommen Pflanzenfette, zum Beispiel gehärtete Pflanzenöle, die Triglyceride von Fettsäuren mit 12-18 Kohlenstoffatomen, vorzugsweise die Trägerstoffe der Markenbezeichnung Witepsol®, in Betracht. Der Wirkstoff wird in der geschmolzenen Trägermasse homogen verteilt und das erhaltene Gemisch zu Suppositorien vergossen.

Zur parenteralen Verabreichung werden Präparate in Injektionslösungsform hergestellt. Dazu werden die Wirkstoffe, gegebenenfalls in Gegenwart von Lösungsmittlern, wie Polyoxyäthylensorbitanmonolaurat, -monooleat oder -monostearat (Tween 20®, Tween 60®, Tween 80®), in destilliertem Wasser und/oder unterschiedlichen organischen Lösungsmitteln, zum Beispiel Glykoläthern, gelöst. Die Injektionslösungen können ferner unterschiedliche Hilfsstoffe, zum Beispiel Konservierungsmittel, wie Benzylalkohol, p-Oxybenzoesäuremethyl- oder -propylester, Benzalkoniumchlorid oder Phenylmercuriborat, Antioxydantien, wie Ascorbinsäure, Tocopherol oder Natriumpyrosulfat, zum Binden von Metallspuren Komplexbildner, z. B. Äthylendiamintetraessigsäure, Puffersubstanzen oder Stoffe zum Einstellen des pH-Wertes sowie gegebenenfalls Lokalanästhetika, wie Lidocain, enthalten. Die Injektionslösungen werden vor dem Abfüllen filtriert und, nachdem sie in Ampullen gefüllt wurden, sterilisiert.

Die Tagesdosis beträgt, abhängend vom Zustand des Kranken, 0,1-300 mg/kg, vorzugsweise 2,0-160 mg/kg, und wird zweckmässig in kleineren Einzeldosen verabreicht.

Die Erfindung wird im folgenden an Hand von Ausführungsbeispielen näher erläutert.

*Beispiel 1:*

*α-Äthyl-α-(2-methoxyphenyl)-4-[2-(diisopropylamino)-äthoxy]-benzylalkohol*

Aus 1,82 g Magnesiumspänen und 14 g 2-Bromanisol wird in 53 ml Tetrahydrofuran ein Grignard-Reagens bereitet und zu diesem unter schwachem Sieden am Rückfluss tropfenweise die mit 30 ml Tetrahydrofuran bereitete Lösung von 14 g 4-[2-(Diisopropylamino)-äthoxy]-propiophenon gegeben. Das Gemisch wird eine weitere Stunde lang eben am Sieden gehalten und nach dem Abkühlen in gesättigte wässerige Ammoniumchloridlösung eingegossen. Das Tetrahydrofuran wird unter vermindertem Druck abdestilliert und der Rückstand mit Benzol extrahiert. Die Lösung wird zuerst mit gesättigter Kochsalzlösung, dann mit Wasser gewaschen, über wasserfreiem Magnesiumsulfat getrocknet und nach dem Filtrieren im Vakuum eingedampft. Der Rückstand wird aus n-Hexan kristallisiert. Man erhält 15 g der Titelverbindung. Schmp.: 46-47° C.

*Elementaranalyse für die Summenformel* $C_{24}H_{35}NO_3$:

| | | | |
|---|---|---|---|
| Berechnet: | C 74,76 | H 9,15 | N 3,63% |
| Gefunden: | C 74,87 | H 9,23 | N 3,77% |

*Beispiel 2:*

*α-Äthyl-α-(4-fluorphenyl)-4-[3-(di-n-propylamino)-propoxy]-benzylalkohol*

Aus 3,6 g Magnesiumspänen und 16,3 g Äthylbromid wird in 60 ml wasserfreiem Äther eine Äthylmagnesiumbromidlösung hergestellt und dieser bei 0-5° C unter Rühren die mit 70 ml wasserfreiem Äther bereitete Lösung von 13,2 g 4-Fluor-4′-[3-(dipropylamino)-propoxy]-benzophenon tropfenweise zugesetzt. Das Reaktionsgemisch wird bei Raumtemperatur 30 Minuten lang gerührt und dann in eine mit Eiswasser bereitete Ammoniumchloridlösung gegossen. Die wässerige Phase wird mit Äther extrahiert, die organischen Phasen werden vereinigt, mit Wasser neutral gewaschen, über wasserfreiem Magnesiumsulfat getrocknet und der Äther unter vermindertem Druck abgedampft. Der Rückstand wird im Vakuum destilliert. Man erhält 11,7 g der Titelverbindung. Siedepunkt: 196-198° C/13 Pa.

*Elementaranalyse für die Summenformel* $C_{24}H_{34}FNO_2$:

| | | | | |
|---|---|---|---|---|
| Berechnet: | C 74,38 | H 8,84 | F 4,90 | N 3,61% |
| Gefunden: | C 74,46 | H 3,87 | F 5,11 | N 3,72% |

Die Base wird in wasserfreiem Äthanol gelöst und die Lösung mit einer äthanolischen Lösung der äquivalenten Menge Fumarsäure versetzt. Aus der auf −10° C gekühlten Lösung wird das Hydrofumarate mit Äther ausgefällt. Das Salz wird abfiltriert, mit Äther gewaschen und dann getrocknet. Schmelzpunkt: 87-89° C.

*Beispiel 3:*

*α-Äthyl-α-(4-chlorphenyl)-4-[3-(di-n-propylamino)-propoxy]-benzylalkohol*

Ein Gemisch aus 13,1 g α-Äthyl-α-(4-chlorphenyl)-4-hydroxy-benzylalkohol, 14 g wasserfreiem Kaliumcarbonat, 9,8 g Dipropylaminopropylchlorid, 0,85 g Tetrabutylammoniumhydrogensulfat und 135 ml Äthylacetat wird unter Rühren 20 Stunden lang eben am Sieden gehalten. Nach dem Abkühlen wird das Lösungsmittel im Vakuum abgezogen. Zu dem Rückstand werden Wasser und Benzol gegeben. Die Phasen werden voneinander getrennt, die organische Phase wird mit Wasser gewaschen, über wasserfreiem Kaliumcarbonat getrocknet und nach dem Filtrieren eingedampft. Der Rückstand wird im Vakuum fraktioniert. Man erhält 13,8 g der Titelverbindung. Siedepunkt: 198-200° C/1,33 Pa.

*Elementaranalyse für die Summenformel* $C_{24}H_{34}ClNO_2$:

| | | | | |
|---|---|---|---|---|
| Berechnet: | C 71,35 | H 8,48 | Cl 8,78 | N 3,47% |
| Gefunden: | C 71,41 | H 8,53 | Cl 8,94 | N 3,59% |

*Beispiel 4:*

*α-Äthyl-α-(2,4-dichlorphenyl)-4-[3-(di-n-propylamino)-propoxy]-benzylalkohol*

21,7 g α-Äthinyl-α-(2,4-dichlorphenyl)-4-[3-(dipropylamino)-propoxy]-benzylalkohol werden in 210 ml Benzol gelöst und nach Zugabe von 1,1 g 10%iger Palladiumaktivkohle hydriert. Nach Aufnahme der berechneten Menge Wasserstoff (etwa 3 Stunden) wird der Katalysator abfiltriert und das Benzol abgezogen. Das rohe Produkt (21 g) wird im Vakuum destilliert. Siedepunkt: 194-196° C/6,6 Pa.

*Elementaranalyse für die Summenformel* $C_{24}H_{33}Cl_2NO_2$:

| | | | | |
|---|---|---|---|---|
| Berechnet: | C 65,74 | H 7,59 | Cl 16,17 | N 3,19% |
| Gefunden: | C 65,83 | H 7,74 | Cl 16,40 | N 3,15% |

*α-Äthyl-α-(2,4-dichlorphenyl)-4-[3-(di-n-propylamino)-propoxy]-benzylalkohol-methojodid*

4,4 g der Base und 12,4 g Methyljodid werden in 22 ml Aceton gelöst. Die Lösung wird am Rückfluss 2 Stunden lang schwach gekocht und dann abgekühlt. Nach Zugabe von Äther fällt das quaternäre Salz kristallin aus, wird abfiltriert und getrocknet. 3,6 g der quarternären Verbindung werden erhalten. Schmelzpunkt: 136-137° C.

*Beispiel 5:*

*α-Äthyl-α-(4-bromphenyl)-4-[3-(di-n-propylamino)-propoxy]-benzylalkohol*

Aus 2,2 g Magnesiumspänen und 28,2 g 4-[3-(Di-n-propylamino)-propoxy]-brombenzol wird in 170 ml wasserfreiem Tetrahydrofuran ein Grignardreagens bereitet und zu diesem bei 20° C Tropfenweise die mit 60 ml Tetrahydrofuran bereitete Lösung von 12,8 g 4-Brompropiophenon gegeben. Das Reaktionsgemisch wird bei 20° C noch 2 Stunden lang gerührt und dann unter Rühren mit 20%iger wässeriger Ammoniumchloridlösung versetzt. Das Tetrahydrofuran wird unter vermindertem Druck abgezogen. Der Rückstand wird mit Benzol extrahiert. Der Extrakt wird mit Wasser neutral gewaschen, über wasserfreiem Magnesiumsulfat getrocknet und nach dem Filtrieren unter vermindertem druck eingedampft. Der Rückstand wird im Vakuum fraktioniert. Man erhält 19,9 g der Titelverbindung. Siedepunkt: 220-224° C/6,6 Pa.

*Elementaranalyse* für $C_{24}H_{34}BrNO_2$:

Berechnet: C 64,28 H 7,64 Br 17,82 N 3,12%

Gefunden: C 64,40 H 7,75 Br 17,94 N 3,28%

Schmelzpunkt des Hydrofumarats: 106-107° C.

*Beispiel 6:*

*α-Äthyl-α-(3-chlorphenyl)-4-[3-(di-n-propylamino)-propoxy]-benzylalkohol*

Ein Gemisch aus 13,1 g α-Äthyl-α-(3-chlorphenyl)-4-hydroxy-benzylalkohol, 22,8 g wasserfreiem Kaliumcarbonat, 0,4 ml 40%igem Tetrabutylammoniumhydroxyd und 130 ml Methylisobutylketon wird zum Sieden erhitzt und mit 11,8 g Di-n-propylaminopropylchlorid-hydrochlorid in 12 ml Wasser versetzt. Das Reaktionsgemisch wird 5 Stunden lang gekocht und dann das Lösungsmittel unter vermindertem Druck abdestilliert. Der Rückstand wird mit Wasser verdünnt und mit Benzol extrahiert. Die organischen Phasen werden vereinigt, mit 5%iger wässeriger Kalilauge und dann mit Wasser neutral gewaschen und über wasserfreiem Magnesiumsulfat getrocknet. Das Lösungsmittel wird unter vermindertem Druck abdestilliert und der Rückstand fraktioniert. Man erhält 13,5 g der Titelverbindung. Siedepunkt: 200-202° C/6,6 Pa.

*Elementaranalyse für die Summenformel* $C_{24}H_{34}ClNO_2$:

Berechnet: C 71,35 H 8,48 Cl 8,78 N 3,47%

Gefunden: C 71,46 H 9,55 Cl 8,88 N 3,52%

Schmelzpunkt des Hydrofumarats: 136-137° C.

*Beispiel 7:*

*α-Äthyl-α-(3-trifluormethylphenyl)-4-[3-(di-n-propylamino)-propoxy]-benzylalkohol*

Ein Gemisch aus 20,8 α-Äthyl-α-(3-trifluormethylphenyl)-4-(3-brompropoxy)-benzylalkohol und 41 ml Dipropylamin wird unter Rühren 6 Stunden lang am Rückfluss schwach gekocht. Nach dem Abkühlen wird das Reaktionsgemisch unter vermindertem Druck eingedampft. Der Rückstand wird mit Wasser versetzt und mit Benzol extrahiert. Die organische Phase wird mit Wasser gewaschen, über wasserfreiem Kaliumcarbonat getrocknet und denn eingedampft. Der Rückstand wird im Vakuum fraktioniert. Man erhält 17,2 g der Titelverbindung. Siedepunkt: 176-178° C/6,6 Pa.

*Elementaranalyse für die Summenformel* $C_{25}H_{34}F_3NO_2$:

Berechnet: C 68,62 H 7,83 F 13,03 N 3,02

Gefunden: C 68,57 H 7,88 F 13,17 N 3,41%

Schmelpunkt des Hydrofumarats: 85-86° C.

Durch entsprechende Wahl der Ausgangsstoffe werden analog zu den obigen Beispielen folgende Verbindungen hergestellt.

*α-Äthyl-α-(3-trifluormethlyphenyl)-4-[2-(diisopropylamino)-äthoxy]-benzylalkohol*

Siedepunkt: 172-174° C/6,6 Pa.

*Elementaranalyse für die Summenformel* $C_{24}H_{32}F_3NO_2$:

Berechnet: C 68,06 H 7,62 F 13,46 N 3,31%

Gefunden: C 68,22 H 7,36 F 13,70 N 3,54%

*α-Äthyl-α-(4-chlorphenyl)-4-[2-(diisopropylamino)-äthoxy]-benzylalkohol*

Siedepunkt: 204-206° C/6,6 Pa.

*Elementaranalyse für die Summenformel* $C_{23}H_{32}Cl_2NO_2$:

Berechnet: C 70,84 H 8,27 Cl 9,09 N 3,59%

Gefunden: C 70,81 H 8,18 Cl 9,26 N 3,77%

*α-Äthyl-α-(2-trifluormethylphenyl)-4-[2-(diisopropylamino)-äthoxy]-benzylalkohol*

Siedepunkt: 197-201° C/20 Pa.

*Elementaranalyse für die Summenformel* $C_{24}H_{32}F_3NO_2$:

Berechnet: C 68,06 H 7,62 F 13,46 N 3,31%

Gefunden: C 67,88 H 7,73 F 13,50 N 3,10%

*α-Äthyl-α-(2-methoxyphenyl)-2-[2-(diisopropylamino)-äthoxy]-benzylalkohol*

Schmelzpunkt: 71-72° C.

*Elementaranalyse für die Summenformel* $C_{24}H_{35}NO_3$:

Berechnet: C 74,76 H 9,15 N 3,63%

Gefunden: C 74,70 H 9,17 N 3,84%

*α-Äthyl-α-(4-fluorphenyl)-4-[2-(diisopropylamino)-äthoxy]-benzylalkohol*

Siedepunkt: 191-194° C/13,3 Pa.

*Elementaranalyse für die Summenformel* $C_{23}H_{32}FNO_2$:

Berechnet: C 73,96 H 8,64 F 5,09 N 3,75%

Gefunden: C 74,23 H 9,80 F 5,34 N 3,67%

*α-Äthyl-α-(2,5-dimethylphenyl)-4-[2-(diisopropylamino)-äthoxy]-benzylalkohol*

Siedepunkt: 200-204° C/13,3 Pa.

*Elementaranalyse für die Summenformel* $C_{25}H_{37}NO_2$:

Berechnet: C 78,28 H 9,72 N 3,65%

Gefunden: C 78,53 H 10,01 N 3,55%

*α-Äthyl-α-(3-chlorphenyl)-4-[2-(diisopropylamino)-äthoxy]-benzylalkohol*

Siedepunkt: 190-192° C/6,6 Pa.

*Elementaranalyse für die Summenformel* $C_{23}H_{32}ClNO_2$:

Berechnet: C 70,84 H 8,27 Cl 9,09 N 3,59%

Gefunden: C 71,10 H 8,52 Cl 9,17 N 3,44%

*α-Äthyl-α-(2-methoxyphenyl)-4-[3-(di-n-propylamino)-propoxy]-benzylalkohol*

Siedepunkt: 212-214° C/6,6 Pa.

*Elementaranalyse für die Summenformel* $C_{25}H_{37}NO_3$:

| | | | |
|---|---|---|---|
| Berechnet: | C 75,15 | H 9,33 | N 3,50% |
| Gefunden: | C 75,23 | H 9,28 | N 3,63% |

Schmelzpunkt des Hydrofumarats: 107-108° C.

*Beispiel 8:*

Aus den erfindungsgemässen Verbindungen können zum Beispiel die folgenden Arzneimittelpräparate hergestellt werden.

*Tabletten*

Zusammensetzung einer Tablette:

| | |
|---|---|
| Wirkstoff | 100,0 mg |
| Lactose | 184,0 mg |
| Kartoffelstärke | 80,0 mg |
| Polyvinylpyrrolidon | 8,0 mg |
| Talk | 12,0 mg |
| Magnesiumstearat | 2,0 mg |
| Aerosil® (kolloidales $SiO_2$) | 2,0 mg |
| Ultraamylopektin | 12,0 mg |

Aus den aufgeführten Stoffen werden durch Nassgranulieren und Pressen Tabletten eines Gewichtes von 400 mg hergestellt.

Wirkstoff: α-Äthyl-α-(2-methoxyphenyl)-4-[3-(di-n-propylamino)-propoxy]-benzylalkohol

*Dragees*

Die auf die beschriebene Weise hergestellten Tabletten werden in an sich bekannter Weise mit einer aus Zucker und Talk bestehenden Schicht überzogen und dann mit einem Gemisch aus Bienenwachs und Carnaubawachs poliert.

*Suppositorien*

Zusammensetzung eines Suppositoriums:

| | |
|---|---|
| Wirkstoff | 100,0 mg |
| Lactose | 200,0 mg |
| Suppositoriengrundmasse (z. B. Witepsol® H) | 1700,0 mg |

Die Grundmasse wird geschmolzen und die Schmelze auf 35° C gekühlt. Der Wirkstoff wird gründlich mit der Lactose vermischt und das Gemisch in einem Homogenisator mit der Schmelze homogenisiert. Das erhaltene Gemisch wird in gekühlte Suppositorienformen gegossen. Gewicht eines Suppositoriums: 2000 mg.

Wirkstoff: α-Äthyl-α-(-3-chlorphenyl)-4-[3-(di-n-propylamino)-propoxy]-benzylalkohol

*Kapseln*

Zusammensetzung der Füllung einer Kapsel:

| | |
|---|---|
| Wirkstoff | 50,0 mg |
| Lactose | 100,0 mg |
| Talk | 2,0 mg |
| Kartoffelstärke | 30,0 mg |
| Mikrokristalline Cellulose | 8,0 mg |

Der Wirkstoff wird gründlich mit den Hilfsstoffen vermischt, das Gemisch durch ein Sieb der Maschenweite 0,32 mm gesiebt und dann im Hartgelatinekapseln der Grösse 4 gefüllt.

Wirkstoff: α-Äthyl-α-(3-chlorophenyl)-4-[3-(di-n-propylamino)-propoxy]-benzylalkohol.

*Suspension*

100 ml Suspension enthalten:

| | |
|---|---|
| Wirkstoff | 1,0 g |
| Natriumhydroxyd | 0,26 g |
| Citronensäure | 0,30 g |
| Nipagin® (Natriumsalz des 4-Hydroxybenzoesäuremethylesters) | 0,10 g |
| Carbopol® 940 (Polyacrylsäure) | 0,30 g |
| Äthanol (96%ig) | 1,00 g |
| Himbeeraroma | 0,60 g |
| Sorbit (70%ige wässerige Lösung) | 71,00 g |
| dest. Wasser zum Auffüllen auf | 100,00 ml |

Das Nipagin® und die Citronensäure werden in 20 ml destilliertem Wasser gelöst. Zu der Lösung gibt man in kleinen Portionen, unter intensivem Rühren das Carbopol® und lässt die Lösung dann 10-12 Stunden lang stehen. Anschliessend werden die mit 1 ml destilliertem Wasser bereitete Lösung des Natriumhydroxyds, dann die wässerige Lösung des Sorbits und schliesslich die äthanolische Lösung des Himbeeraromas unter Rühren zugegeben. Zu der so bereiteten Trägersubstanz gibt man in kleinen Portionen den Wirkstoff und homogenisiert das Ganze mit einem Mixer. Die Suspension wird mit dest. Wasser auf 100 ml aufgefüllt und in einer Kolloidmühle homogenisiert.

Wirkstoff: α-Äthyl-α-(2-trifluormethylphenyl)-4-[3-(diisopropylamino)-äthoxy]-benzylalkohol.

**Patentansprüche** für die Vertragsstaaten BE, CH, DE, FR, GB, IT, LI, LU, NL, SE

1. Substituierte α-[Äthyl]-α-[phenyl]-(ω-[dialkylamino)-alkoxy]-benzylalkohole der allgemeinen Formel

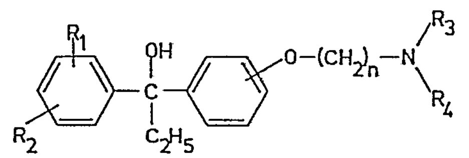

I,

worin

$R_1$ für ein Wasserstoffatom, ein Halogenatom, einen Trihalogenmethylrest oder einen geradkettigen oder verzweigten Alkyl- oder Alkoxyrest mit je 1 bis 4 Kohlenstoffatom(en) steht,

$R_2$ ein Halogenatom, einen Trihalogenmethylrest oder einen geradkettigen oder verzweigten Alkyl- oder Alkoxyrest mit je 1 bis 4 Kohlenstoffatom(en) bedeutet,

$R_3$ und $R_4$ gleiche oder verschiedene geradkettige oder verzweigte Alkylreste mit 3 bis 5 Kohlenstoffatomen darstellen, und

n eine ganze Zahl von 2 bis 5 ist,

sowie ihre Säureadditionssalze und quaternären Ammoniumsalze.

2. α-[Äthyl]-α-[phenyl]-[ω-(dialkylamino)-alkoxy]-benzylalkohole nach Anspruch 1, dadurch gekennzeichnet, dass das, beziehungsweise die, Halogenatom(e), für das, beziehungsweise die, $R_1$ und/oder $R_2$ stehen kann, beziehungsweise können, [ein] Chlor-, Fluor- und/oder Bromatom(e) ist, beziehungsweise sind.

3. α-[Äthyl]-α-[phenyl]-[ω-(dialkylamino)-alkoxy]-benzylalkohole nach Anspruch 1 oder 2, dadurch gekennzeichnet, dass der, beziehungsweise die, Trihalogenmethylrest(e), für den, beziehungsweise die, $R_1$ und/oder $R_2$ stehen kann, beziehungsweise können, [ein] Trifluormethylrest(e) und/oder Trichlormethylrest(e) ist, beziehungsweise sind.

4. α-[Äthyl]-α-[phenyl]-[ω-(dialkylamino)-alkoxy]-benzylalkohole nach Anspruch 1 bis 3, dadurch gekennzeichnet, dass der, beziehungsweise die, Alkyl- und/oder Alkoxyrest(e), für den, beziehungsweise die, $R_1$ und/oder $R_2$ stehen kann, beziehungsweise können, ein solcher, beziehungsweise solche, mit 1 oder 2, insbesondere 1, Kohlenstoffatom(en) ist, beziehungsweise sind.

5. α-[Äthyl]-α-[phenyl]-[ω-(dialkylamino)-alkoxy]-benzylalkohole nach Anspruch 1 bis 4, dadurch gekennzeichnet, dass die Alkylreste, für die $R_3$ und $R_4$ stehen, solche mit 3 oder 4, insbesondere 3, Kohlenstoffatomen sind.

6. α-[Äthyl]-α-[phenyl]-[ω-(dialkylamino)-alkoxy]-benzylalkohole nach Anspruch 1 bis 5, dadurch gekennzeichnet, dass n 2 bis 4, insbesondere 2 oder 3 ist.

7. Die α-[Äthyl]-α-[phenyl]-[ω-(dialkylamino)-alkoxy]-benzylalkohole α-Äthyl-α-(2-methoxyphenyl)-4-[2-(diisopropylamino)-äthoxy]-benzylalkohol, α-Äthyl-α-(4-fluorphenyl)-4-[3-(di-n-propylamino)-propoxy]-benzylalkohol, α-Äthyl-α-(4-chlorphenyl)-4-[3-(di-n-propylamino)-propoxy]-benzylalkohol, α-Äthyl-α-(2,4-dichlorphenyl)-4-[3-(di-n-propylamino)-propoxy]-benzylalkohol, α-Äthyl-α-(4-bromphenyl)-4-[3-(di-n-propylamino)-propoxy]-benzylalkohol, α-Äthyl-α-(3-chlorphenyl)-4-[3-(di-n-propylamino)-propoxy]-benzylalkohol, α-Äthyl-α-(3-trifluormethylphenyl)-4-[3-(di-n-propylamino)-propoxy]-benzylalkohol, α-Äthyl-α-(3-trifluormethylphenyl)-4-[2-(diisopropylamino)-äthoxy]-benzylalkohol, α-Äthyl-α-(4-chlorphenyl)-4-[2-(diisopropylamino)-äthoxy]-benzylalkohol, α-Äthyl-α-(2-trifluormethylphenyl)-4-[2-(diisopropylamino)-äthoxy]-benzylalkohol, α-Äthyl-α-(2-methoxyphenyl)-2-[2-(diisopropylamino)-äthoxy]-benzylalkohol, α-Äthyl-α-(4-fluorphenyl)-4-[2-(diisopropylamino)-äthoxy]-benzylalkohol, α-Äthyl-α-(2,5-dimethylphenyl)-4-[2-(diisopropylamino)-äthoxy]-benzylalkohol, α-Äthyl-α-(3-chlorphenyl)-4-[2-(diisopropylamino)-äthoxy]-benzylalkohol und α-Äthyl-α-(2-methoxyphenyl)-4-[3-(di-n-propylamino)-propoxy]-benzylalkohol sowie ihre Salze.

8. Verfahren zur Herstellung der Verbindungen nach Anspruch 1 bis 7, dadurch gekennzeichnet, dass man

a) am Benzolring ω-(dialkylamino)-alkoxysubstituierte Propiophenone der allgemeinen Formel

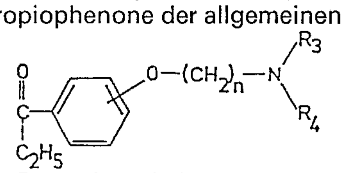

II,

worin $R_3$, $R_4$ und n wie im Anspruch 1, 5 oder 6 festgelegt sind, mit eine Phenylgruppe aufweisenden metallorganischen Verbindungen der allgemeinen Formel

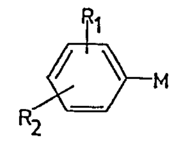

III,

worin

$R_1$ und $R_2$ wie in den Ansprüchen 1 bis 4 festgelegt sind, und

M für ein Alkalimetallatom, vorzugsweise Lithium-, Natrium- oder Kaliumatom, oder einen Rest der Formel MgX mit X = Halogenatom steht, umsetzt oder

b) substituierte α-[Äthyl]-α-[phenyl]-oder ω-(halogen)-alkoxy]-benzylalkohole der allgemeinen Formel

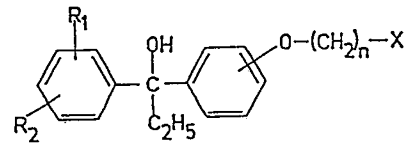

IV,

worin

$R_1$, $R_2$ und n wie in den Ansprüchen 1 bis 4 oder 6 festgelegt sind, und

X für ein Halogenatom steht,

mit Dialkylaminen der allgemeine Formel

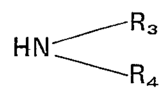

V,

worin $R_3$ und $R_4$ wie im Anspruch 1 oder 5 festgelegt sind, vorzugsweise in Gegenwart von Basen, umsetzt oder

c) substituierte [ω-(Dialkylamino)-alkoxy]-benzophenone der allgemeinen Formel

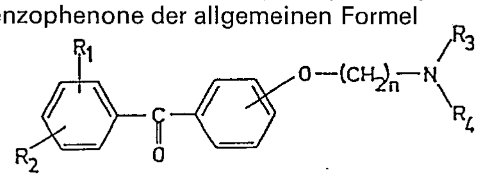

VI,

worin $R_1$, $R_2$, $R_3$, $R_4$ und n wie in den Ansprüchen 1 bis 6 festgelegt sind, mit eine Äthylgruppe aufweisenden metallorganischen Verbindungen, insbesondere Äthylmagnesiumhalogeniden oder Äthyllithium, umsetzt, oder

d) am Benzolring substituierte Propiophenone der allgemeinen Formel

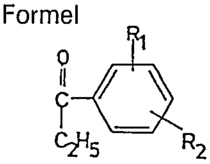

VII,

worin $R_1$ und $R_2$ wie in den Ansprüchen 1 bis 4 festgelegt sind, mit eine {[ω-(Dialkylamino)-alkoxy]-phenyl}-gruppe aufweisenden Grignard-Verbindungen der allgemeinen Formel

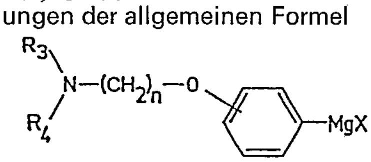

VIII,

worin

$R_3$, $R_4$ und n wie im Anspruch 1, 5 oder 6 festgelegt sind, und

X für ein Halogenatom steht,
umsetzt, oder
e) substituierte 1-[Phenyl]-1-[hydroxyphenyl]-propan-1-ole der allgemeinen Formel

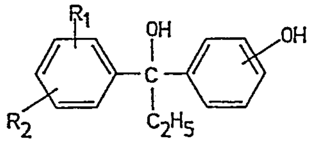
IX

worin $R_1$ und $R_2$ wie in den Ansprüchen 1 bis 4 festgelegt sind, vorzugsweise in Form ihrer Alkaliphenolate oder quaternären Ammoniumphenolate, mit tertiären Aminen der allgemeinen Formel

$$X-(CH_2)_n-N{<}^{R_3}_{R_4} \qquad X,$$

worin
$R_3$, $R_4$ und n wie im Anspruch 1, 5 oder 6 festgelegt sind, und
X für ein Halogenatom oder einen Alkylsulfonyloxy- oder Arylsulfonyloxyrest steht,
oder mit deren Salzen, vorzugsweise in Gegenwart von säurebindenden Mitteln, umsetzt, oder
f) substituierte α-[Äthinyl]- beziehungsweise α-[Vinyl]-α-[phenyl]-[ω-(dialkylamino)-alkoxy]-benzylalkohole der allgemeinen Formel

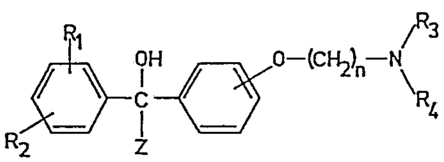
XI,

worin
$R_1$, $R_2$, $R_3$, $R_4$ und n wie in den Ansprüchen 1 bis 6 festgelegt sind, und
Z für einen Äthinyl- oder Vinylrest steht,
reduziert,
worauf man gegebenenfalls in an sich bekannter Weise die erhaltenen α-[Äthyl]-α-[phenyl]-[ω-(dialkylamino)-alkoxy]-benzylalkohole der allgemeinen Formel I mit Säuren in Säureadditionssalze oder mit Quaternisierungsmitteln in quaternäre Ammoniumsalze überführt oder gegebenenfalls die erhaltenen Säureadditionssalze der α-[Äthyl]-α-[phenyl]-[ω-(dialkylamino)-alkoxy]-benzylalkohole der allgemeinen Formel I in die entsprechenden freien Basen der allgemeinen Formel I und/oder in andere Säureadditionssalze überführt.

9. Arzneimittel, gekennzeichnet durch einen Gehalt an 1 oder mehr Verbindung(en) nach Anspruch 1 bis 7 als Wirkstoff(e), zweckmässigerweise zusammen mit 1 oder mehr üblichen pharmazeutischen Konfektionierungsmittel(n).

**Patentansprüche** für den Vertragsstaat: AT

1. Verfahren zur Herstellung von substituierten α-[Äthyl]-α-[phenyl]-[ω-(dialkylamino)-alkoxy]-benzylalkoholen der allgemeinen Formel

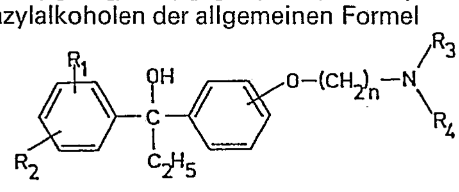
I,

worin
$R_1$ für ein Wasserstoffatom, ein Halogenatom, einen Trihalogenmethylrest oder einen geradkettigen oder verzweigten Alkyl- oder Alkoxyrest mit je 1 bis 4 Kohlenstoffatom(en) steht,
$R_2$ ein Halogenatom, einen Trihalogenmethylrest oder einen geradkettigen oder verzweigten Alkyl- oder Alkoxyrest mit je 1 bis 4 Kohlenstoffatom(en) bedeutet,
$R_3$ und $R_4$ gleiche oder verschiedene geradkettige oder verzweigte Alkylreste mit 3 bis 5 Kohlenstoffatomen darstellen, und
n eine ganze Zahl von 2 bis 5 ist,
sowie ihren Säureadditionssalzen und quaternären Ammoniumsalzen, dadurch gekennzeichnet, dass man
a) am Benzolring ω-(dialkylamino)-alkoxysubstituierte Propiophenone der allgemeinen Formel

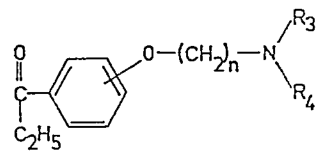
II,

worin $R_3$, $R_4$ und n wie oben festgelegt sind, mit eine Phenylgruppe aufweisenden metallorganischen Verbindungen der allgemeinen Formel

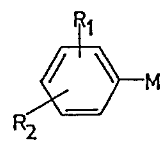
III,

worin
$R_1$ und $R_2$ wie oben fesgelegt sind, und
M für ein Alkalimetallatom, vorzugsweise Lithium-, Natrium- oder Kaliumatom, oder einen Rest der Formel MgX mit X = Halogenatom steht,
umsetzt, oder
b) substituierte α-[Äthyl]-α-[phenyl]-[ω-(halogen)-alkoxy]-benzylalkohole der allgemeinen Formel

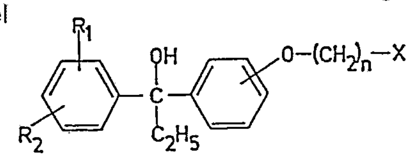
IV,

worin
$R_1$, $R_2$ und n wie oben festgelegt sind, und
X für ein Halogenatom steht,
mit Dialkylaminen der allgemeinen Formel

$$HN{<}^{R_3}_{R_4} \qquad V,$$

worin $R_3$ und $R_4$ wie oben festgelegt sind, vorzugsweise in Gegenwart von Basen, umsetzt, oder
c) substituierte [ω-(Dialkylamino)-alkoxy]-benzophenone der allgemeinen Formel

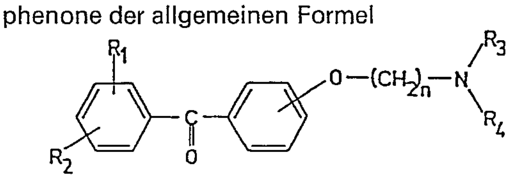
VI,

worin $R_1$, $R_2$, $R_3$, $R_4$ und n wie oben festgelegt

sind, mit eine Äthylgruppe aufweisenden metallorganischen Verbindungen, insbesondere Äthylmagnesiumhalogeniden oder Äthyllithium, umsetzt, oder

d) am Benzolring substituierte Propiophenone der allgemeinen Formel

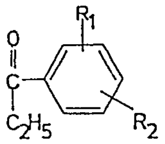

VII,

worin $R_1$ und $R_2$ wie oben festgelegt sind, mit eine {[ω-(Dialkylamino)-alkoxy]-phenyl}-gruppe aufweisenden Grignard-Verbindungen der allgemeinen Formel

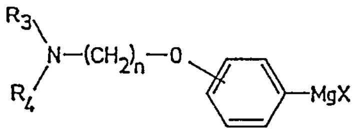

VIII,

worin

$R_3$, $R_4$ und n wie oben festgelegt sind, und

X für ein Halogenatom steht,

umsetzt, oder

e) substituierte 1-[Phenyl]-1-[hydroxyphenyl]-propan-1-ole der allgemeinen Formel

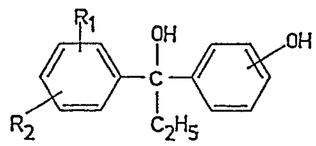

IX,

worin $R_1$ und $R_2$ wie oben festgelegt sind, vorzugsweise in Form ihrer Alkaliphenolate oder quaternären Ammoniumphenolate, mit tertiären Aminen der allgemeinen Formel

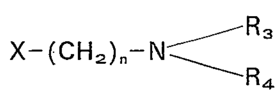

X,

worin

$R_3$, $R_4$ und n wie oben festgelegt sind, und

X für ein Halogenatom oder einen Alkylsulfonyloxy- oder Arylsulfonyloxyrest steht,

oder mit deren Salzen, vorzugsweise in Gegenwart von säurebindenden Mitteln, umsetzt, oder

f) substituierte α-[Äthinyl]- beziehungsweise α-[Vinyl]-α-[phenyl]-[ω-(dialkylamino)-alkoxy]-benzylalkohole der allgemeinen Formel

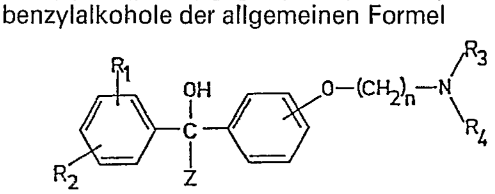

XI,

worin

$R_1$, $R_2$, $R_3$, $R_4$ und n wie oben festgelegt sind, und

Z für einen Äthinyl- oder Vinylrest steht,

reduziert,

worauf man gegebenenfalls in an sich bekannter Weise die erhaltenen α-[Äthyl]-α-[phenyl]-[ω-(dialkylamino)-alkoxy]-benzylalkohole der allgemeinen Formel I mit Säuren in Säureadditionssalze oder mit Quaternisierungsmitteln in quaternäre Ammoniumsalze überführt oder gegebenenfalls die erhaltenen Säureadditionssalze der α-[Äthyl]-α-[phenyl]-[ω-(dialkylamino)-alkoxy]-benzylalkohole der allgemeinen Formel I in die entsprechenden freien Basen der allgemeinen Formel I und/oder in andere Säureadditionssalze überführt.

2. Verfahren nach Anspruch 1, dadurch gekennzeichnet, dass man α-[Äthyl]-α-[phenyl]-[ω-(dialkylamino)-alkoxy]-benzylalkohole, bei welchen das, beziehungsweise die, Halogenatom(e), für das, beziehungsweise die, $R_1$ und/oder $R_2$ stehen kann, beziehungsweise können, [ein] Chlor-, Fluor- und/oder Bromatom(e) ist, beziehungsweise sind, herstellt.

3. Verfahren nach Anspruch 1 oder 2, dadurch gekennzeichnet, dass man α-[Äthyl]-α-[phenyl]-[ω-(dialkylamino)-alkoxy]-benzylalkohole, bei welchen der, beziehungsweise die, Trihalogenmethylrest(e), für den, beziehungsweise die, $R_1$ und/oder $R_2$ stehen kann, beziehungsweise können, [ein] Trifluormethylrest(e) und/oder Trichlormethylrest(e) ist, beziehungsweise sind, herstellt.

4. Verfahren nach Anspruch 1 bis 3, dadurch gekennzeichnet, dass man α-[Äthyl]-α-[phenyl]-[ω-(dialkylamino)-alkoxy]-benzylalkohole, bei welchen der, beziehungsweise die, Alkyl- und/oder Alkoxyrest(e), für den, beziehungsweise die, $R_1$ und/oder $R_2$ stehen kann, beziehungsweise können, ein solcher, beziehungsweise solche, mit 1 oder 2, insbesondere 1, Kohlenstoffatom(en) ist, beziehungsweise sind, herstellt.

5. Verfahren nach Anspruch 1 bis 4, dadurch gekennzeichnet, dass man α-[Äthyl]-α-[phenyl]-[ω-(dialkylamino)-alkoxy]-benzylalkohole, bei welchen die Alkylreste, für die $R_3$ und $R_4$ stehen, solche mit 3 oder 4, insbesondere 3, Kohlenstoffatomen sind, herstellt.

6. Verfahren nach Anspruch 1 bis 5, dadurch gekennzeichnet, dass man α-[Äthyl]-α-[phenyl]-[ω-(dialkylamino)-alkoxy]-benzylalkohole, bei welchen n 2 bis 4, insbesondere 2 oder 3, ist, herstellt.

7. Verfahren nach Anspruch 1 bis 6, dadurch gekennzeichnet, dass man die α-[Äthyl]-α-[phenyl]-[ω-(dialkylamino)-alkoxy]-benzylalkohole α-Äthyl-α-(2-methoxyphenyl)-4-[2-(diisopropylamino)-äthoxy]-benzylalkohol, α-Äthyl-α-(4-fluorphenyl]-4-[3-(di-n-propylamino)-propoxy]-benzylalkohol, α-Äthyl-α-(4-chlorphenyl)-4-[3-(di-n-propylamino)-propoxy]-benzylalkohol, α-Äthyl-α-(2,4-dichlorphenyl)-4-[3-(di-n-propylamino)-propoxy]-benzylalkohol, α-Äthyl-α-(4-bromphenyl)-4-[3-(di-n-propylamino)-propoxy)-benzylalkohol, α-Äthyl-α-(3-chlorphenyl)-4-[3-(di-n-propylamino)-propoxy]-benzylalkohol, α-Äthyl-α-(3-trifluormethylphenyl)-4-[3-(di-n-propylamino)-propoxy]-benzylalkohol, α-Äthyl-α-(3-trifluormethylphenyl)-4-[2-(diisopropylamino)-äthoxy]-benzylalkohol, α-Äthyl-α-(4-chlorphenyl)-4-[2-(diisopropylamino)-äthoxy]-benzylalkohol, α-Äthyl-α-(2-trifluormethylphenyl)-4-[2-(diisopropylamino)-äthoxy]-benzylalkohol, α-Äthyl-α-(2-methoxyphenyl)-2-[2-(diisopropylamino)-äthoxy]-benzylalkohol, α-Äthyl-α-(4-

fluorphenyl)-4-[2-(diisopropylamino)-äthoxy]-benzylalkohol, α-Äthyl-α-(2,5-dimethylphenyl)-4-[2-(diisopropylamino)-äthoxy]-benzylalkohol, α-Äthyl-α-(3-chlorphenyl)-4-[2-(diisopropylamino)-äthoxy]-benzylalkohol und α-Äthyl-α-(2-methoxyphenyl)-4-[3-(di-n-propylamino)-propoxy]-benzylalkohol sowie ihre Salze herstellt.

**Claims** for the Contracting States BE, CH, DE, FR, GB, IT, LI, LU, NL, SE

1. Substituted α-[ethyl]-α-[phenyl]-[ω-(dialkylamino)-alkoxy]-benzyl alcohols of the generic formula

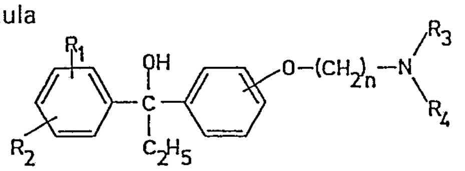

I,

wherein

$R_1$ stands for a hydrogen atom, a halogen atom, a trihalogenmethyl radical or a straight or branched alkyl- or alkoxy radical having each 1 to 4 carbon atom(s),

$R_2$ means a halogen atom, a trihalogenmethyl radical or a straight or branched alkyl- od alkoxy radical having each 1 to 4 carbon atom(s),

$R_3$ und $R_4$ are same or different straight or branched alkyl radicals having 3 to 5 carbon atoms, and

n is an integer of 2 to 5

and their acid addition salts and quaternary ammonium salts.

2. α-[Ethyl]-α-[phenyl]-[ω-(dialkylamino)-alkoxy]-benzyl alcohols according to claim 1, characterized in that the halogen atom(s) for which $R_1$ and/or $R_2$ can stand is (are) [a] chlorine-, fluorine- and/or bromine atom(s).

3. α-[Ethyl]-α-[phenyl]-[ω-(dialkylamino)-alkoxy]-benzyl alcohols according to claim 1 or 2, characterized in that the trihalogenmethyl radical(s) for which $R_1$ and/or $R_2$ can stand is (are) [a] trifluoromethyl radical(s) and/or trichloromethyl radical(s).

4. α-[Ethyl]-α-[phenyl]-[ω-(dialkylamino)-alkoxy]-benzyl alcohols according to claims 1 to 3, characterized in that the alkyl- and/or alkoxy radical(s) for which $R_1$ and/or $R_2$ can stand is such one (are those ones) having 1 or 2, especially 1, carbon atom(s).

5. α-[Ethyl]-α-[phenyl]-[ω-(dialkylamino)-alkoxy]-benzyl alcohols according to claims 1 to 4, characterized in that the alkyl radicals for which $R_3$ and $R_4$ are standing are those having 3 or 4, especially 3, carbon atoms.

6. α-[Ethyl]-α-[phenyl]-[ω-(dialkylamino)-alkoxy]-benzyl alcohols according to claims 1 to 5, characterized in that n is 2 to 4, especially 2 or 3.

7. The α-[ethyl]-α-[phenyl]-[ω-(dialkylamino)-alkoxy]-benzyl alcohols α-ethyl-α-(2-methoxyphenyl)-4-[2-(diisopropylamino)-ethoxy]-benzyl alcohol, α-ethyl-α-(4-fluorophenyl)-4-[3-(di-n-propylamino)-propoxy]-benzyl alcohol, α-ethyl-α-(4-chlorophenyl)-4-[3-(di-n-propylamino)-propoxy]-benzyl alcohol, α-ethyl-α-(2,4-dichlorophenyl)-4-[3-(di-n-propylamino)-propoxy]-benzyl alcohol, α-ethyl-α-(4-bromophenyl)-4-[3-(di-n-propylamino)-propoxy]-benzyl alcohol, α-ethyl-α-(3-chlorophenyl)-4-[3-(di-n-propylamino)-propoxy]-benzyl alcohol, α-ethyl-α-(3-trifluormethylphenyl)-4-[-3-(di-n-propylamino)-propoxy]-benzyl alcohol, α-ethyl-α-(3-trifluormethylphenyl)-4-[2-(diisopropylamino)-ethoxy]-benzyl alcohol, α-ethyl-α-(4-chlorophenyl)-4-[2-(diisopropylamino)-ethoxy]-benzyl alcohol, α-ethyl-α-(2-trifluoro- methylphenyl)-4-[2-(diisopropylamino)-ethoxy]-benzyl alcohol, α-ethyl-α-(2-methoxyphenyl)-2-[2-(diisopropylamino)-ethoxy]-benzyl alcohol, α-ethyl-α-(4-fluorophenyl)-4-[2-(diisopropylamino)-ethoxy]-benzyl alcohol, α-ethyl-α-(2,5-dimethylphenyl)-4-[2-(diisopropylamino)-ethoxy]-benzyl alcohol, α-ethyl-α-(3-chlorophenyl)-4-[2-(diisopropylamino)-ethoxy]-benzyl alcohol and α-ethyl-α-(2-methoxyphenyl)-4-[3-(di-n-propylamino)-propoxy]-benzyl alcohol and their salts.

8. A process for preparing the compounds according to claims 1 to 7, characterized in that

a) propiophenones ω-(dialkylamino)-alkoxy-substituted on the benzene ring of the generic formula

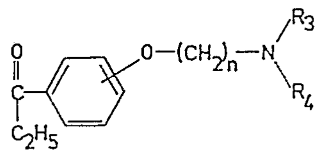

II,

wherein $R_3$, $R_4$ and n are as defined in claim 1, 5 or 6, are reacted with organometallic compounds having a phenyl group of the generic formula

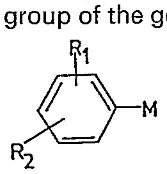

III,

wherein

$R_1$ and $R_2$ are as defined in claims 1 to 4, and

M stands for an alkali metal atom, preferably lithium-, sodium- or potassium atom, or a radical of the formula MgX, X being a halogen atom, or

b) substituted α-[ethyl]-α-[phenyl]-[ω-(dialkylamino)-alkoxy]-benzyl alcohols of the generic formula

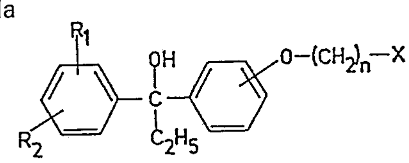

IV,

wherein $R_1$, $R_2$ and n are as defined in the claims 1 to 4 or 5, and

X stands for a halogen atom,

are reacted with dialkyl amines of the generic formula

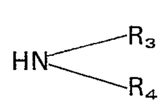

V,

wherein $R_3$ and $R_4$ are as defined in claim 1 or 5, preferably in the presence of bases, or

c) substituted [ω-(dialkylamino)-alkoxy]benzophenones of the generic formula

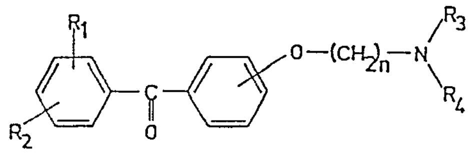

VI,

wherein $R_1$, $R_2$, $R_3$, $R_4$ and n are as defined in the claims 1 to 6, are reacted with an organometallic compound having an ethyl group, especially ethyl magnesium halides or ethyllithium, or

d) propiophenones substituted on the benzene ring of the generic formula

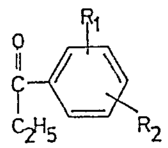

VII,

wherein $R_1$ and $R_2$ are as defined in the claims 1 to 4, are reacted with a Grignard-compound having a {[ω-(dialkylamino)-alkoxy]-phenyl}-group of the generic formula

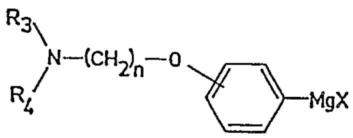

VIII,

wherein $R_3$, $R_4$ and n are as defined in claim 1, 5 or 6, and

X being a halogen atom, or

e) substituted 1-[phenyl]-1-[hydroxyphenyl]-propane-1-oles of the generic formula

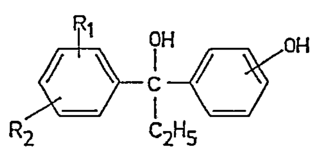

IX,

wherein $R_1$ and $R_2$ are as defined in the claims 1 to 4, preferably in form of their alkali phenolates or quaternary ammonium phenolates, are reacted with tertiary amines of the generic formula

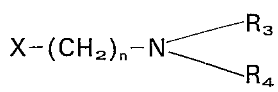

X,

wherein

$R_3$, $R_4$ and n are as defined in claim 1, 5 or 6, and

X being a halogen atom or an alkylsulfonyloxy- or arylsulfonyloxy radical,

or with their salts, preferably in the presence of acid binding agents, or

f) substitutes α-[ethinyl]- and α-[vinyl]-α-[phenyl]-[ω-(dialkylamio)-alkoxy]-benzyl alcohols, respectively of the generic formula

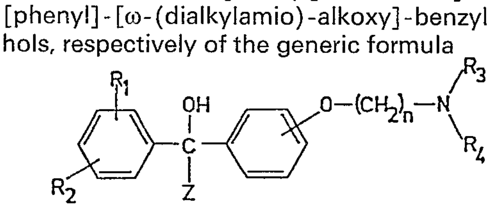

XI,

wherein $R_1$, $R_2$, $R_3$, $R_4$ and n are as defined in claims 1 to 6, and

Z being an ethinyl- or vinyl radical,

are reduced,

and subsequentyl optionally in a manner known per se the obtained α-[ethyl]-α-[phenyl]-[ω-(dialkylamino)-alkoxy]-benzyl alcohol of the generic Formula I are converted with acids into acid addition salts or with quaternizing agents into quaternary ammonium salts or optionally the obtained acid addition salts of the α-[ethyl]-α-[phenyl]-[ω-(dialkylamino)-alkoxy]-benzyl alcohols of the generic Formula I are converted into the corresponding free bases of the generic Formula I and/or in different acid addition salts.

9. Pharmaceutical, characterized by a content of 1 or more compound(s) according to claims 1 to 7 as active ingredient(s), preferably together with 1 or more conventional pharmaceutical auxiliary agent(s).

**Claims** for the Contracting State: AT

1. A process for preparing substituted α-[ethyl]-α-[phenyl]-[ω-(dialkylamino)-alkoxy]-benzyl alcohols of the generic formula

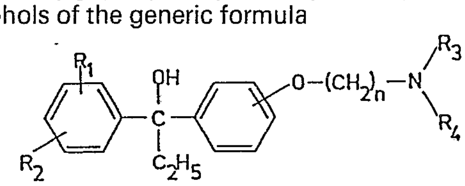

I,

wherein

$R_1$ stands for a hydrogen atom, a halogen atom, a trihalogenmethyl radical or a straight or branched alkyl- or alkoxy radical having each 1 to 4 carbon atom(s),

$R_2$ means a halogen atom, a trihalogenmethyl radical or a straight or branched alkyl- or alkoxy radical having each 1 to 4 carbon atom(s),

$R_3$ and $R_4$ are same or different straight or branched alkyl radicals having 3 to 5 carbon atoms, and

n is an integer of 2 to 5

and their acid addition salts and quaternary ammonium salts, characterized in that

a) propiophenones ω-(dialkylamino)-alkoxy-substituted on the benzene ring of the generic formula

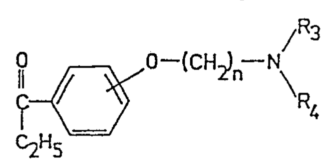

II,

wherein $R_3$, $R_4$ and n are as defined above, are reacted with organometallic compounds having a phenyl group of the generic formula

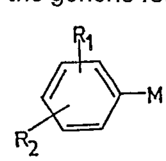

III,

wherein $R_1$ and $R_2$ are as defined above, and

M stands for an alkali metal atom, preferably lithium-, sodium- or potassium atom, or a radical of the formula MgX, X being a halogen atom, or

b) substituted α-[ethyl]-α-[phenyl]-[ω-(halogen)-alkoxy]-benzyl alcohols of the generic formula

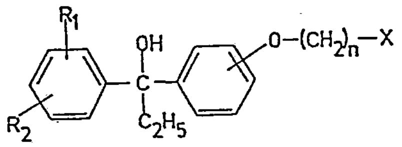
IV,

wherein $R_1$, $R_2$ and n are as defined above, and
X stands for a halogen atom,
are reacted with dialkyl amines of the generic formula

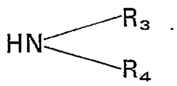
V,

wherein $R_3$ and $R_4$ are as defined above, preferably in the presence of bases, or
c) substituted [ω-(dialkylamino)-alkoxy]-benzophenones of the generic formula

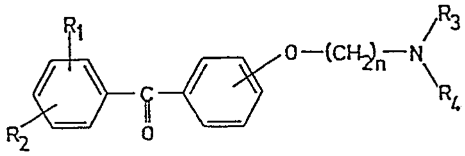
VI,

wherein $R_1$, $R_2$, $R_3$, $R_4$ and n are as defined above, are reacted with an organometallic compound having an ethyl group, especially ethyl magnesium halides or ethyllithium, or
d) propiophenones substituted on the benzene ring of the generic formula

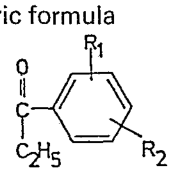
VII,

wherein $R_1$ and $R_2$ are as defined above, are reacted with a Grignard-compound having a {[ω-(dialkylamino)-alkoxy]-phenyl}-group of the generic formula

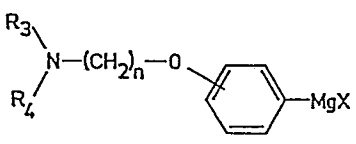
VIII,

wherein
$R_3$, $R_4$ and n are as defined above, and
X being a halogen atom, or
e) substituted 1-[phenyl]-1-[hydroxyphenyl]-propane-1-oles of the generic formula

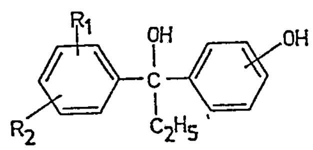
IX,

wherein $R_1$ und $R_2$ are as defined above, preferably in form of their alkali phenolates or quaternary ammonium phenolates, are reacted with tertiary amines of the generic formula

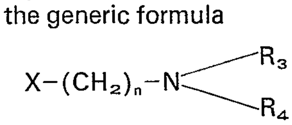
X,

wherein
$R_3$, $R_4$ and n are as defined above, and
X being a halogen atom or an alkylsulfonyloxy- or arylsulfonyloxy radical,
or with their salts, preferably in the presence of acid binding agents, or
f) substitutes α-[ethinyl]- and α-[vinyl]-α-[phenyl]-[ω-(dialkylamino)-alkoxy]-benzyl alcohols, respectively of the generic formula

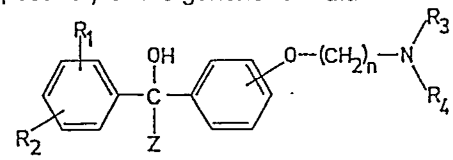
XI,

wherein
$R_1$, $R_2$, $R_3$, $R_4$ and n are as defined above, and
Z being an ethinyl- or vinyl radical, are reduced, and subsequently optionally in a manner known per se the obtained α-[ethyl]-α-[phenyl]-[ω-(dialkylamino)-alkoxy]-benzyl alcohols of the generic formula I are converted with acids into acid addition salts or with quaternizing agents into quaternary ammonium salts or optionally the obtained acid addition salts of the α-[ethyl]-α-[phenyl]-[ω-(dialkylamino)-alkoxy]-benzyl alcohols of the generic formula I are converted into the corresponding free bases of the generic formula I and/or in different acid addition salts.

2. A process according to claim 1, characterized in that α-[ethyl]-α-[phenyl]-[ω-(dialkylamino)-alkoxy]-benzyl alcohols are prepared in which the halogen atom(s) for which $R_1$ and/or $R_2$ can stand is (are) [a] chlorine-, fluorine- and/or bromine atom(s).

3. A process according to claim 1 or 2, characterized in that α-[ethyl]-α-[phenyl]-[ω-(dialkylamino)-alkoxy]-benzyl alcohols are prepared in which the trihalogenmethyl radical(s) for which $R_1$ and/or $R_2$ can stand is (are) [a] trifluoromethyl radical(s) and/or trichloromethyl radical(s).

4. A process according to claims 1 to 3, characterized in that α-[ethyl]-α-[phenyl]-[ω-(dialkylamino)-alkoxy]-benzyl alcohols are prepared in which the alkyl- and/or alkoxy radical(s) for which $R_1$ and/or $R_2$ can stand, is such one (are those ones) having 1 or 2, especially 1, carbon atom(s).

5. A process according to claims 1 to 4, characterized in that α-[ethyl]-α-[phenyl]-[ω-(dialkylamino)-alkoxy]-benzyl alcohols are prepared, in which the alkyl radicals for which $R_3$ and $R_4$ are standing are those ones having 3 or 4, especially 3, carbon atoms.

6. A process according to claims 1 to 5, characterized in that α-[ethyl]-α-[phenyl]-[ω-(dialkylamino)-alkoxy]-benzyl alcohols are prepared, in which n is 2 to 4, especially 2 or 3.

7. A process according to claims 1 to 6, characterized in that the α-[ethyl]-α-[phenyl]-[ω-(dialkylamino)-alkoxy]-benzyl alcohols α-[ethyl]-α-(2-methoxyphenyl)-4-[2-(diisopropylamino)-ethoxy]-benzyl alcohol, α-ethyl-α-(4-fluorophenyl)-4-[3-(di-n-propylamino)-propoxy]-benzyl alcohol, α-ethyl-α-(4-chlorophenyl)-4-[3-(di-n-propylamino)-propoxy]-benzyl alcohol, α-ethyl-α-(2,4-dichlorophenyl)-4-[3-(di-n-propylamino)-propoxy]-benzyl alcohol, α-ethyl-α-(4-bromophenyl)-4-[3-(di-n-propylamino)-propoxy]-benzyl alcohol, α-ethyl-α-(3-chlorophenyl)-4-[3-(di-n-propylamino)-propoxy]-benzyl alcohol, α-ethyl-α-(3-trifluoromethylphenyl)-4-[3-(di-n-propylamino)-propoxy]-benzyl alcohol, α-ethyl-α-(3-trifluoromethylphenyl)-4-[2-(diisopropylamino)-ethoxy]-benzyl alcohol, α-ethyl-α-(4-chlorophenyl)-4-[2-(diisopropylamino)-ethoxy]-benzyl alcohol, α-ethyl-α-(2-trifluoro-methylphenyl)-4-[2-(diisopropylamino)-ethoxy]-benzyl alcohol, α-ethyl-α-(2-methoxyphenyl)-2-[-2-(diisopropylamino)-ethoxy]-benzyl alcohol, α-ethyl-α-(4-fluorophenyl)-4-[2-(diisopropylamino)-ethoxy]-benzyl alcohol, α-ethyl-α-(2,5-dimethylphenyl)-4-[2-(diisopropylamino)-ethoxy]-benzyl alcohol, α-ethyl-α-(3-chlorophenyl)-4-[-2-(diisopropylamino)-ethoxy]-benzyl alcohol and α-ethyl-α-(2-methoxyphenyl)-4-[3-(di-n-propylamino)-propoxy]-benzyl alcohol and their salts are prepared.

**Revendications** pour les Etats contractants BE, CH, DE, FR, GB, IT, LI, LU, NL, SE

1. Alcools α-[éthyl]-α-[phényl]-[ω-(dialcoylamino)-alcoxy]-benzyliques substitués, répondant à la formule générale:

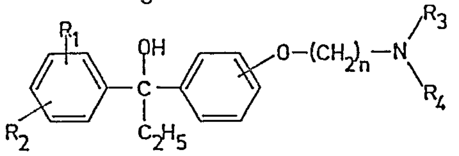
I

dans laquelle

$R_1$ représente un atome d'hydrogène, un atome d'halogène, un reste trihalométhyle ou bien un reste alcoyle ou alcoxy linéaire ou ramifié comportant, dans chaque cas, un à quatre atome(s) de carbone,

$R_2$ représente un atome d'halogène, un reste trihalométhyle ou bien un reste alcoyle ou alcoxy linéaire ou ramifié comportant, dans chaque cas, un à quatre atome(s) de carbone,

$R_3$ et $R_4$ représentent des restes alcoyle linéaires ou ramifiés identiques ou différents, comportant 3 à 5 atomes de carbone, et

n est un nombre entier de 2 à 5

ainsi que leurs sels d'addition avec des acides et leurs sels d'ammonium quaternaires.

2. Alcools α-[éthyl]-α-[phényl]-[ω-(dialcoylamino)-alcoxy]-benzyliques selon la revendication 1, caractérisés en ce que [le ou les atome(s) d'halogène, que peut ou peuvent représenter $R_1$ et/ou $R_2$, est/sont, un (des) atome(s) de chlore, de fluor et/ou de brome].

3. Alcools α-[éthyl]-α-[phényl]-[ω-(dialcoylamino)-alcoxv]-benzyliques selon la revendication 1 ou 2, caractérisés en ce que [le ou les reste(s) trihalométhyle que peut ou peuvent représenter $R_1$ et/ou $R_2$, est/sont, un (des) reste(s) trifluorométhyle et/ou un (des) reste(s) trichlorométhyle].

4. Alcools α-[éthyl]-α-[phényl]-[ω-(dialcoylamino)-alcoxy]-benzyliques selon les revendications 1 à 3, caractérisés en ce que [le ou les reste(s) alcoyle et/ou alcoxy que peut ou peuvent représenter $R_1$ et/ou $R_2$, est/sont un reste ou des restes comportant 1 ou 2 atome(s) de carbone, en particulier 1 atome de carbone].

5. Alcools α-[éthyl]-α-[phényl]-[ω-(dialcoylamino)-alcoxy]-benzyliques selon les revendications 1 à 4, caractérisés en ce que [les restes alcoyle que représentent $R_3$ et $R_4$ sont des restes comportant 3 ou 4, notamment 3 atomes de carbone].

6. Alcools α-[éthyl]-α-[phényl]-[ω-(dialcoylamino)-alcoxy]-benzyliques selon les revendications 1 à 5, caractérisés en ce que [n est égal à un nombre de 2 à 4, notamment à 2 ou à 3].

7. Alcools α-[éthyl]-α-[phényl]-[ω-(dialcoylamino)-alcoxy]-benzyliques suivants:
les alcools α-éthyl-α-(2-méthoxyphényl)-4-[2-(di-isopropylamino)-éthoxy]-benzylique, α-éthyl-α-(4-fluorophényl)-4-[3-(di-n-propylamino)-propoxy]-benzylique, α-éthyl-α-(4-chlorophényl)-4-[3-(di-n-propylamino)-propoxy]-benzylique, α-éthyl-α-(2,4-dichlorophényl)-4-[3-(di-n-propylamino)-propoxy]-benzylique, α-éthyl-α-(4-bromophényl)-4-[-3-(di-n-propylamino)-propoxy]-benzylique, α-éthyl-α-(3-chlorophényl)-4-[3-(di-n-propylamino)-propoxy]-benzylique, α-éthyl-α-(3-trifluorométhylphényl)-4-[3-(di-n-propylamino)-propoxy]-benzylique, α-éthyl-α-(3-trifluorométhylphényl)-4-[2-(di-isopropylamino)-éthoxy]-benzylique, α-éthyl-α-(4-chlorophényl)-4-[2-(di-isopropylamino)-éthoxy]-benzylique, α-éthyl-α-(2-trifluorométhylphényl)-4-[2-(di-isopropylamino)-éthoxy]-benzylique, α-éthyl-α-(2-méthoxyphényl)-2-[2-(di-isopropylamino)-éthoxy]-benzylique, α-éthyl-α-(4-fluorophényl)-4-[2-(di-isopropylamino)-éthoxy]-benzylique, α-éthyl-α-(2,5-diméthylphényl)-4-[2-(di-isopropylamino)-éthoxy]-benzylique, α-éthyl-α-(3-chlorophényl)-4-[2-(di-isopropylamino)-éthoxy]-benzylique et α-éthyl-α-(2-méthoxyphényl)-4-[3-(di-n-propylamino)-propoxy]-benzylique, ainsi que leurs sels.

8. Procédé de préparation des composés selon les revendications 1 à 7, caractérisé en ce que

a) l'on fait réagir des phénones propioniques à substitution ω-(dialcoylamino)-alcoxy sur le noyau benzénique, répondant à la formule générale:

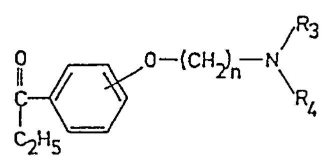
II

dans laquelle $R_3$, $R_4$ et n ont la même signification que dans la revendication 1, 5 ou 6, sur des composés organométalliques comportant un groupe phényle répondant à la formule générale:

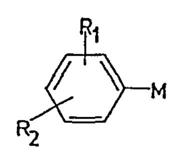
III

dans laquelle

$R_1$ et $R_2$ ont la même signification que dans les revendications 1 à 4, et

M représente un atome de métal alcalin, de préférence un atome de lithium, de sodium ou de potassium, ou un reste répondant à la formule MgX, X étant un atome d'halogène, ou bien

b) l'on fait réagir des alcools α-[éthyl]-α-[phényl]-[ω-(halo)-alcoxy]-benzyliques substitués répondant à la formule générale:

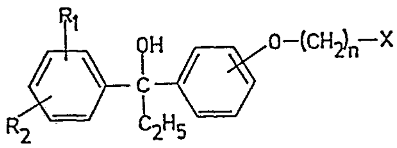

IV

dans laquelle

$R_1$, $R_2$ et n ont la même signification que dans les revendications 1 à 4 ou 6, et

X représente un atome d'halogène,

sur des dialcoylamines répondant à la formule générale:

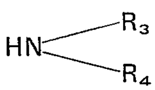

V

dans laquelle $R_3$ et $R_4$ ont la même signification que dans la revendication 1 ou 5, de préférence en présence de bases, ou bien

c) on fait réagir des [ω-(dialcoylamino)-alcoxy]-benzophénones substituées répondant à la formule générale:

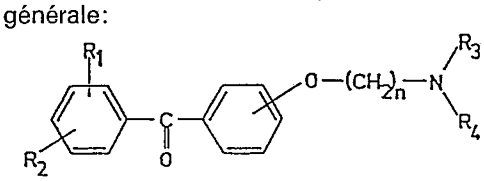

VI

dans laquelle $R_1$, $R_2$, $R_3$, $R_4$ et n ont la même signification que dans les revendications 1 à 6, sur des composés organométalliques comportant un groupe éthyle, notamment des halogénures d'éthyl-magnésium ou d'éthyl-lithium, ou bien

d) on fait réagir des phénones propioniques substituées sur le noyau benzénique répondant à la formule générale:

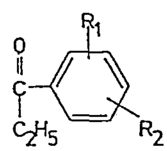

VII

dans laquelle $R_1$ et $R_2$ ont la même signification que dans les revendications 1 à 4, sur des composés de Grignard comportant un groupe {[ω-(dialcoylamino)-alcoxy]-phényle} répondant à la formule générale:

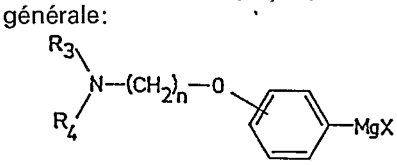

VIII

dans laquelle

$R_3$, $R_4$ et n ont la même signification que dans la revendication 1, 5 ou 6, et

X représente un atome d'halogène,

ou bien

e) on fait réagir des 1-[phényl]-1-[hydroxyphényl]-propane-1-oles substitués répondant à la formule générale:

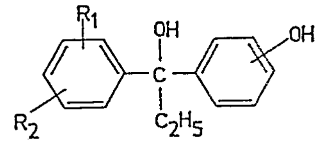

IX

dans laquelle $R_1$ et $R_2$ ont la même signification que dans les revendications 1 à 4, de préférence sous la forme de leurs phénolates alcalins ou de leurs phénolates d'ammonium quaternaires, sur des amines tertiaires répondant à la formule générale:

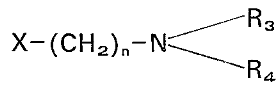

X

dans laquelle

$R_3$, $R_4$ et n ont la même signification que dans la revendication 1, 5 ou 6, et

X représente un atome d'halogène ou bien un reste alcoylsulfonyloxy ou arylsulfonyloxy,

ou sur leurs sels, de préférence en présence d'agents de fixation des acides, ou bien

f) on réduit des alcools α-[éthynyl]- ou α-[vinyl]-α-[phényl]-[ω-(dialcoylamino]-alcoxy]-benzyliques répondant à la formule générale:

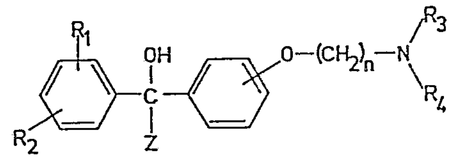

XI

dans laquelle

$R_1$, $R_2$, $R_3$, $R_4$ et n ont la même signification que dans les revendications 1 à 6, et

Z représente un reste éthynyle ou vinyle,

et l'on transforme alors éventuellement, de façon connue en soi, les alcools α-[éthyl]-α-[phényl]-[ω-(dialcoylamino)-alcoxy]-benzyliques obtenus, répondant à la formule générale I, avec des acides, en sels d'addition avec des acides, ou bien, avec des agents de transformation en dérivés quaternaires ou «quaternisation», en sels d'ammonium quaternaires, ou bien, l'on transforme les sels d'addition avec des acides des alcools α-[éthyl]-α-[phényl]-[ω-(dialcoylamino)-alcoxy]-benzyliques obtenus en les bases libres correspondantes répondant à la formule générale I et/ou en d'autres sels d'addition avec des acides.

9. Médicament, caractérisé en ce qu'il renferme un ou plusieurs composé(s) selon les revendications 1 à 7, comme principe(s) actif(s), de façon appropriée en même temps qu'un ou plusieurs agent(s) de confection pharmaceutique(s) usuel(s).

**Revendications** pour l'Etat contractant: AT

1. Procédé de préparation des composés répondant à la formule générale:

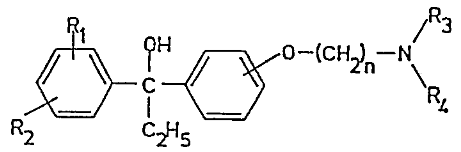

I

dans laquelle

$R_1$ représente un atome d'hydrogène, un atome d'halogène, un reste trihalométhyle ou bien un reste alcoyle ou alcoxy linéaire ou ramifié comportant, dans chaque cas, un à quatre atome(s) de carbone,

$R_2$ représente un atome d'halogène, un reste trihalométhyle ou bien un reste alcoyle ou alcoxy linéaire ou ramifié comportant, dans chaque cas, un à quatre atome(s) de carbone,

$R_3$ et $R_4$ représentent des restes alcoyle linéaires ou ramifiés identiques ou différents, comportant 3 à 5 atomes de carbone, et

n est un nombre entier de 2 à 5

ainsi que leurs sels d'addition avec des acides et leurs sels d'ammonium quaternaires, caractérisé en ce que

a) l'on fait réagir des phénones propioniques à substitution ω-(dialcoylamino)-alcoxy sur le noyau benzénique, répondant à la formule générale:

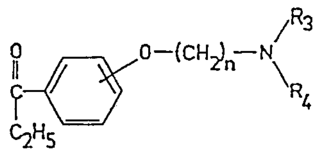

II

dans laquelle $R_3$, $R_4$ et n ont la même signification que dans la revendication 1, 5 ou 6, sur des composés organométalliques comportant un groupe phényle répondant à la formule générale:

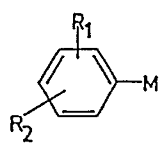

III

dans laquelle

$R_1$ et $R_2$ ont la même signification que dans les revendications 1 à 4, et

M représente un atome de métal alcalin, de préférence un atome de lithium, de sodium ou de potassium, ou un reste répondant à la formule MgX, X étant un atome d'halogène, ou bien

b) l'on fait réagir des alcools α-[éthyl]-α-[phényl]-[ω-(halo)-alcoxy]-benzyliques substitués répondant à la formule générale:

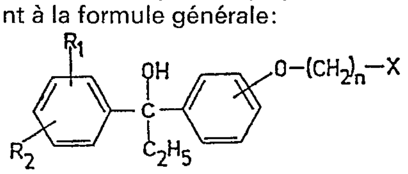

IV

dans laquelle

$R_1$, $R_2$ et n ont la même signification que dans les revendications 1 à 4 ou 6, et

X représente un atome d'halogène,

sur des dialcoylamines répondant à la formule générale:

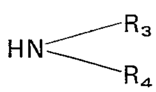

V

dans laquelle $R_3$ et $R_4$ ont la même signification que dans la revendication 1 ou 5, de préférence en présence de bases, ou bien

c) on fait réagir des [ω-(dialcoylamino)-alcoxy]-benzophénones substitués répondant à la formule générale:

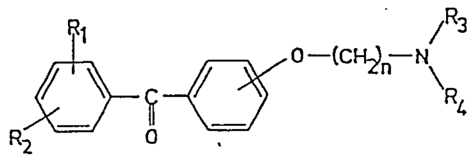

VI

dans laquelle $R_1$, $R_2$, $R_3$, $R_4$ et n ont la même signification que dans les revendications 1 à 6, sur des composés organométalliques comportant un groupe éthyle, notamment des halogénures d'éthyl-magnésium ou d'éthyl-lithium, ou bien

d) on fait réagir des phénones propioniques substituées sur le noyau benzénique répondant à la formule générale:

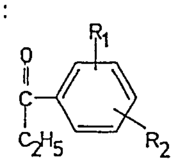

VII

dans laquelle $R_1$ et $R_2$ ont la même signification que dans les revendications 1 à 4, sur des composés de Grignard comportant un groupe {[ω-(dialcoylamino)-alcoxy]-phényle} répondant à la formule générale:

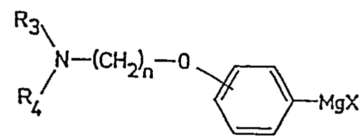

VIII

dans laquelle

$R_3$, $R_4$ et n ont la même signification que dans la revendication 1, 5 ou 6, et

X représente un atome d'halogène,

ou bien

e) on fait réagir des 1-[phényl]-1-[hydroxyphényl]-propane-1-oles substitués répondant à la formule générale:

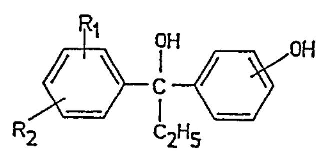

IX

dans laquelle $R_1$ et $R_2$ ont la même signification que dans les revendications 1 à 4, de préférence sous la forme de leurs phénolates alcalins ou de leurs phénolates d'ammonium quaternaires, sur des amines tertiaires répondant à la formule générale:

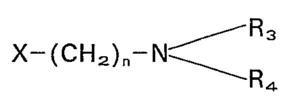

X

dans laquelle

$R_3$, $R_4$ et n ont la même signification que dans la revendication 1, 5 ou 6, et

X représente un atome d'halogène ou bien un reste alcoylsulfonyloxy ou arylsulfonyloxy,

ou sur leurs sels, de préférence en présence d'agents de fixation des acides, ou bien

f) on réduit des alcools α-[éthynyl]-ou α-[vinyl]-α-[phényl]-[ω-(dialcoylamino)-alcoxy]-benzyliques répondant à la formule générale:

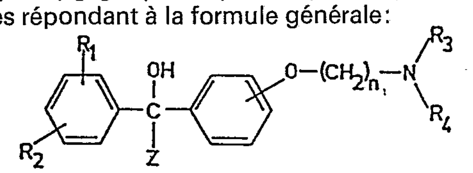
XI

dans laquelle

$R_1$, $R_2$, $R_3$, $R_4$ et n ont la même signification que dans les revendications 1 à 6, et

Z représente un reste éthynyle ou vinyle,

et l'on transforme alors éventuellement, de façon connue en soi, les alcools α-[éthyl]-α-[phényl]-[ω-(dialcoylamino)-alcoxy]-benzyliques obtenus, répondant à la formule générale I, avec des acides, en sels d'addition avec des acides, ou bien, avec des agents de transformation en dérivés quaternaires ou «quaternisation», en sels d'ammonium quaternaires, ou bien, l'on transforme les sels d'addition avec des acides des alcools α-[éthyl]-α-[phényl]-[ω-(dialcoylamino)-alcoxy]-benzyliques obtenus en les bases libres correspondantes répondant à la formule générale I et/ou en d'autres sels d'addition avec des acides.

2. Procédé selon la revendication 1, caractérisé en ce que des alcools α-[éthyl]-α-[phényl]-[ω-(dialcoylamino)-alcoxy]-benzyliques sont préparés dans lesquels le ou les atome(s) d'halogène, que peut ou peuvent représenter $R_1$ et/ou $R_2$, est/sont, un (des) atome(s) de chlor, de fluor et/ou de brome.

3. Procédé selon la revendication 1 ou 2, caractérisé en ce que des alcools α-[éthyl]-α-[phényl]-[ω-(dialcoylamino)-alcoxy]-benzyliques sont préparés dans lesquels le ou les reste(s) trihalométhyle que peut ou peuvent représenter $R_1$ et/ou $R_2$, est/sont, un (des) reste(s) trifluorométhyle et/ou un (des) reste(s) trichlorométhyle.

4. Procédé selon la revendication 1 à 3, caractérisé en ce que des alcools α-[éthyl]-α-[phényl]-[ω-(dialcoylamino)-alcoxy]-benzyliques sont préparés dans lesquels le ou les reste(s) alcoyle et/ou alcoxy que peut ou peuvent représenter $R_1$ et/ou $R_2$, est/sont un reste ou des restes comportant 1 ou 2 atome(s) de carbone, en particulier 1 atome de carbone.

5. Procédé selon la revendication 1 à 4, caractérisé en ce que des alcools α-[éthyl]-α-[phényl]-[ω-(dialcoylamino)-alcoxy]-benzyliques sont préparés dans lesquels les restes alcoyle que représentent $R_3$ et $R_4$ sont des restes comportant 3 ou 4, notamment 3 atomes de carbone.

6. Procédé selon la revendication 1 à 5, caractérisé en ce que des alcools α-[éthyl]-α-[phényl]-[ω-(dialcoylamino)-alcoxy]-benzyliques sont préparés dans lesquels n est égal à un nombre de 2 à 4, notamment à 2 ou à 3.

7. Procédé selon la revendication 1 à 6, caractérisé en ce que les alcools α-[éthyl]-α-[phényl]-[ω-(dialcoylamino)-alcoxy]-benzyliques suivants ainsi que leurs sels sont préparés:

les alcools α-éthyl-α-(2-méthoxyphényl)-4-[2-(di-isopropylamino)-éthoxy]-benzylique, α-éthyl-α-(4-fluorophényl)-4-[3-(di-n-propylamino)-propoxy]-benzylique, α-éthyl-α-(4-chlorophényl)-4-[3-(di-n-propylamino)-propoxy]-benzylique, α-éthyl-α-(2,4-dichlorophényl)-4-[3-(di-n-propylamino)-propoxy]-benzylique, α-éthyl-α-(4-bromophényl)-4-[3-(di-n-propylamino)-propoxy]-benzylique, α-éthyl-α-(3-chlorophényl)-4-[3-(di-n-propylamino)-propoxy]-benzylique, α-éthyl-α-(3-trifluorométhylphényl)-4-[3-(di-n-propylamino)-propoxy]-benzylique, α-éthyl-α-(3-fluorométhylphényl)-4-[2-(di-isopropylamino)-éthoxy]-benzylique, α-éthyl-α-(4-chlorophényl)-4-[2-(di-isopropylamino)-éthoxy]-benzylique, α-éthyl-α-(2-trifluorométhylphényl)-4-[2-(di-isopropylamino)-éthoxy]-benzylique, α-éthyl-α-(2-méthoxyphényl)-2-[2-(di-isopropylamino)-éthoxy]-benzylique, α-éthyl-α-(4-fluorophényl)-4-[2-(di-isopropylamino)-éthoxy]-benzylique, α-éthyl-α-(2,5-diméthylphényl)-4-[2-(di-isopropylamino)-éthoxy]-benzylique, α-éthyl-α-(3-chlorophényl)-4-[2-(di-isopropylamino)-éthoxy]-benzylique et α-éthyl-α-(2-méthoxyphényl)-4-[3-(di-n-propylamino)-propoxy]-benzylique.